# FASCICULE DE BREVET EUROPEEN

(11) **EP 2 387 435 B1**
(45) Date de publication et mention de la délivrance du brevet: **06.07.2016**
(21) Numéro de dépôt: 09797077.6
(22) Date de dépôt: 24.11.2009
(51) Int. Cl.: A61M 39/28, A61M 39/14

(54) **PINCEMENT DE FERMETURE D'UN TUBE SOUPLE DESTINÉ À DES APPLICATIONS BIOPHARMACEUTIQUES**
ZUSAMMENQUETSCHEN EINER FLEXIBLEN RÖHRE ZUR BIOPHARMAZEUTISCHEN VERWENDUNG
PINCHING CLOSED A COLLAPSIBLE TUBE FOR BIOPHARMACEUTICAL USE

(30) Priorité: 10.12.2008 FR 0806952
(43) Date de publication de la demande: 23.11.2011
(73) Titulaire: SARTORIUS STEDIM FMT SAS, 13400 Aubagne (FR)
(72) Inventeur: GAY, Isabelle Valérie Christine, 13124 Peypin (FR); BATES, Michael, Gloucester Gloucestershire GL3 4PD (GB); BASSETTS, Ray, Glos Gloucestershire GL5 4NY (GB); BIDDEL, Chris, Gloucestershire GL10 3LH (GB)
(74) Mandataire: Derambure, Christian
(86) Numéro de dépôt international: PCT/FR2009/052284
(87) Numéro de publication internationale: WO 2010/066978

(56) Documents cités:
- EP-A1- 0 623 450
- EP-A2- 0 778 123
- FR-A1- 2 323 939
- FR-A1- 2 492 261
- FR-A1- 2 688 285
- US-A- 3 588 034
- US-A- 4 688 753
- US-A- 5 238 218

## Description

L'invention a pour objet un dispositif 1 de pincement de fermeture d'un tube souple dans une région de pincement et, selon une réalisation, un ensemble de pincement de fermeture et de sectionnement d'un tel tube.

Dans une réalisation typique dans le domaine biopharmaceutique, un tel tube a un diamètre extérieur compris entre par exemple 8 millimètres et 30 millimètres, l'épaisseur dépendant du matériau, du diamètre et des applications.

Le plus souvent, soit un tel tube fait partie d'un dispositif comportant une ou plusieurs poches ou des moyens fonctionnels (filtration, mélange...), soit il est monté sur, et associé fonctionnellement à, un appareil ou dispositif de traitement (prélèvement, circulation...).

Dans certains cas, le processus concernant le fluide canalisé par le tube nécessite à un moment ou un autre que le tube soit fermé dans une région donnée afin d'empêcher le passage du fluide. Le cas échéant, le processus nécessite que le tube soit non seulement fermé mais également sectionné transversalement dans ou du moins au voisinage de la région de fermeture.

On connaît des pinces de fermeture de tubes souples utilisées dans le domaine biopharmaceutique, connues sous le nom de pinces Halkey ou de pinces Roberts ou encore de pinces Halkey-Roberts. Une telle pince comporte une pièce en matière plastique déformable de forme générale annulaire, ayant deux parties extrêmes libres d'accrochage réciproque en vis-à-vis, un passage pour le tube à l'opposé d'elles, deux saillies dirigées vers la partie médiane de la pince chacune entre une partie d'accrochage et le passage. Une fois la pince placée sur un tube puis fermée par coopération des deux parties d'accrochage, les deux saillies viennent pincer le tube placé entre elles dans une zone de pincement ponctuel ou quasi ponctuel. On peut se référer par exemple aux documents US 3 942 228, US 6 089 527 et US 6 742 760. De telles pinces sont adaptées à un usage temporaire et réversible, mais ne conviennent pas pour un usage permanent et irréversible.

Le document US 4 688 753 décrit un dispositif de pincement d'un tube souple dans une région de pincement, comprenant deux parties à empreinte mâle et femelle, pouvant se trouver soit à l'état ouvert où elles sont écartées l'une de l'autre soit à l'état fermé où elles sont rapprochées et fermées l'une sur l'autre par des moyens de verrouillage réciproque et où les faces des empreintes forment un passage de pincement du tube. Bien que le document US 4 688 753 ne décrive pas les moyens de verrouillage, il prévoit que ceux-ci sont réversibles, le dispositif de pincement étant conçu pour pouvoir être fermé et ouvert à plusieurs reprises. Un tel dispositif n'est pas à usage unique. Le document US 4 688 753 décrit des empreintes mâle et femelle qui, pour leur partie active de pincement, ont des formes spécifiques aplaties avec des bords latéraux divergents dont l'écartement est alors plus grand que le double de l'épaisseur de la paroi du tube souple à pincer, de sorte que le tube n'est pas parfaitement fermé sur lui-même avec le risque de passage de fluide dans la région de pincement.

Le document FR 2 688 285 décrit un dispositif d'obturation d'un tuyau de vidange de poche d'urostomie qui comporte un manchon cylindrique, une pièce d'appui déformable sur le tuyau souple pour réduire sa section interne de passage de fluide et une bague montée à rotation sur le manchon. La rotation de la bague autour du manchon provoque le rapprochement des extrémités de la pièce d'appui déformable qui a une forme allongée, et par suite l'appui de la partie centrale sur le tuyau souple jusqu'à l'écrasement de celui-ci. Le but de ce dispositif est de pouvoir contrôler aisément l'écoulement du fluide dans le tuyau lors de l'ouverture.

Le document US 7 410 155 décrit une pince pour pincer un tube déformable destiné à des applications biopharmaceutiques qui comporte une première section et une seconde section articulées entre elles, chaque section ayant elle-même deux parties articulées entre elles. La première et de la seconde section sont agencées de manière à pouvoir être déplacées à pivotement de façon relative l'une par rapport à l'autre entre une position ouverte et une position fermée. Les quatre parties comportent une empreinte en creux à fond plat venant sur le tube de part et d'autre pour le pincer. La pince comporte également des moyens de verrouillage réciproque des deux parties de chaque paire de parties des deux sections pour les maintenir au moins temporairement en position fermée. Un tel dispositif est destiné à être sectionné pour pouvoir sectionner le tube. Une pince de cette structure présente nombre d'inconvénients. L'usage de charnières ne permet pas une compression uniforme du tube. Le pincement est obtenu par une compression à plat avec le risque de fuite inhérent. La pince n'est pas adaptée à des tubes de différentes géométries (diamètre et/ou épaisseur).

Le document EP 0623450 A1 décrit un appareil de restauration de la forme d'un tube déformé pour permettre un écoulement fluidique dans le tube.

Le document EP 0778123 A2 décrit un dispositif permettant de sectionner un tube préalable écrasé à plat. Les remarques développées ci-avant concernant le document US 7 410 155 s'appliquent également à l'égard de ce document.

Le document US 5238218 A décrit une pince pour obstruer un tube dans laquelle le pincement est obtenu par une compression à plat qui présente les inconvénients pré-cités.

Les documents FR 2492261 A1 et US 3588034 A décrivent des raccords présentant des dispositifs de diminution de section dans lesquels le pincement est obtenu par une compression dans une ouverture de section semi-circulaire. Une telle compression présente un risque de fuite inhérent dans la région de pincement et n'est pas adaptée à des tubes de différentes géométries (diamètre et/ou épaisseur).

Le document FR 2323939 A1 décrit un régulateur de débit comportant une empreinte femelle dans laquelle un téton axial mâle vient comprimer élastiquement un tube de façon réversible et sans déformation plastique du tube. Le but de ce dispositif est de pouvoir contrôler aisément l'écoulement du fluide dans le tuyau lors de l'ouverture.

L'invention vise à proposer un dispositif de pincement de fermeture qui soit à usage unique et remplisse une fonction de fermeture permanente du tube sur lui-même dans la région de pincement empêchant tout passage du fluide dans le tube, ce que les dispositifs selon les documents de l'état de la technique ne permettent pas. En effet, l'exigence d'une fermeture empêchant tout passage du fluide dans le tube est d'autant plus important que la fermeture est permanente, en tout cas n'a pas vocation à être réversible, cette exigence étant moins cruciale dans le cas d'une fermeture temporaire et réversible.

L'invention vise à proposer un tel dispositif qui soit à la fois peu coûteux, aisé à fabriquer, simple et sûr à utiliser. En effet, un tel dispositif doit pouvoir être mis en place sans difficulté mais de manière efficace dans le cours du processus concernant le fluide canalisé par le tube.

A cet effet, selon un premier aspect, l'invention propose un dispositif de pincement de fermeture d'un tube souple dans une région de pincement, comprenant deux sections, chacune ayant une partie creuse à empreinte mâle et femelle, respectivement, pouvant se trouver soit à l'état ouvert où elles sont écartées l'une de l'autre soit à l'état fermé où elles sont rapprochées et fermées l'une sur l'autre par des moyens de verrouillage réciproque et où les faces des empreintes forment un passage de pincement positif du tube.

Ce dispositif est tel que :
- l'empreinte mâle a en section droite transversale une forme de U avec une âme et deux ailes, l'empreinte femelle a en section droite transversale une forme de U avec une âme et deux ailes et le passage de pincement a en section droite transversale une forme de U avec une âme et deux ailes,
- les deux ailes en regard de chaque couple d'ailes en regard de l'empreinte mâle et de l'empreinte femelle vont en se rapprochant l'une de l'autre vers leurs extrémités opposées aux âmes des empreintes, au moins dans la zone de ces extrémités et chacune des deux ailes du passage de pincement a, entre les faces des deux empreintes une ouverture dont la largeur va en diminuant vers son extrémité opposée à l'âme du passage de pincement,
- la largeur de l'ouverture du passage de pincement est au plus légèrement plus petite que le double de l'épaisseur de la paroi du tube souple à pincer.

Le dispositif de pincement de fermeture est à usage unique et remplit une fonction de fermeture du tube sur lui-même dans la région de pincement empêchant tout passage du fluide dans le tube.

Selon une autre caractéristique, les moyens de verrouillage sont irréversibles, les deux sections, une fois amenées à l'état fermé, étant maintenues dans cet état par les moyens de verrouillage sans pouvoir être dissociées, ces moyens de verrouillage comprenant des dents d'accrochage (ou analogue) profilées, orientées selon deux directions opposées.

Selon une première réalisation, les deux sections sont deux pièces distinctes et sont amenées à l'état fermé par un mouvement relatif de coulissement transversal par rapport à l'axe longitudinal du dispositif.

Selon une possibilité, l'une des deux sections est une section mâle et l'autre une section femelle, la section mâle comportant et étant limitée extérieurement par une première âme et deux premières rives, la section femelle comportant et étant limitée extérieurement par une seconde âme et deux secondes rives.

Selon une possibilité, l'écartement entre les deux faces extérieures opposées des deux premières rives est en correspondance avec l'écartement entre les deux faces intérieures en regard des deux secondes rives, de manière à permettre, à l'état fermé du dispositif, l'emboîtement avec ajustement des deux sections, la couple de faces extérieures étant alors en contact et en appui sur la couple de faces intérieures.

Selon une possibilité, les moyens de verrouillage réciproque sont deux ensembles comprenant une série de dents ménagées respectivement sur les deux faces extérieures des premières rives et les deux faces intérieures des secondes rives dans leur zone d'emboîtement.

Selon une possibilité, la section mâle définit une empreinte femelle et la section femelle définit une empreinte mâle.

Selon une seconde réalisation, les deux sections sont deux pièces associées structurellement l'une à l'autre à pivotement autour d'un axe qui, par rapport à l'axe longitudinal du dispositif est déporté sur un côté longitudinal du dispositif, les deux sections étant amenées à l'état fermé par un mouvement relatif de pivotement autour de l'axe de pivotement.

Selon une possibilité, les moyens de verrouillage réciproque comprennent des dents situées du côté opposé à l'axe de pivotement des deux sections.

Selon une caractéristique, les sections sont profilées et s'étendent le long de l'axe longitudinal du dispositif, la zone de pincement positif de fermeture s'étendant sur une certaine longueur axiale, n'étant pas une zone de pincement ponctuel ou quasi ponctuel. Selon une possibilité, les moyens de verrouillage réciproque comprennent une pièce femelle rapportée sur l'une des parties de la section et pourvue de dents et une pièce mâle rapportée sur l'autre des parties et pourvue de dents.

Selon une caractéristique, les deux sections sont de même longueur axiale.

Selon une première variante, les deux empreintes ont une même forme, une même disposition et de mêmes dimensions, constantes et uniformes, d'une extrémité libre à l'autre.

Selon une seconde variante, l'une ou/et l'autre des deux empreintes est légèrement inclinée le long de l'axe longitudinal du dispositif, vers le bord libre de chant de la rive et vers l'extrémité proximale, pour exercer un pincement du tube plus important vers l'extrémité proximale et expulser le fluide se trouvant dans le tube vers l'extrémité distale.

Selon une possibilité, le passage de pincement est d'ouverture réglable, le mouvement relatif pour amener une section en regard d'une autre et le dispositif à l'état fermé étant plus ou moins grand, pour que le passage de pincement soit respectivement, plus ou moins petit. Selon une réalisation possible, le dispositif comprend une série de dents d'accrochage étagées, permettant différentes positions relatives de verrouillage des sections, pour que le passage de pincement soit plus ou moins petit.

Selon une réalisation envisageable, chaque section comporte deux parties creuses placées dans le prolongement l'une de l'autre par des moyens d'association, une couple au moins des parties étant à empreintes.

Selon les exécutions, les deux parties creuses de chaque section sont analogues, le dispositif assurant une fonction de fermeture du tube sur lui-même dans deux zones de pincement positif de fermeture, ou au contraire, les deux parties creuses sont différentes, le dispositif assurant une fonction de fermeture du tube sur lui-même dans une seule zone de pincement positif de fermeture et étant seulement maintenu dans l'autre zone.

Selon les exécutions, les moyens de verrouillage sont irréversibles pour les deux couples de parties creuses, ou irréversibles pour l'une des couples de parties creuses et réversibles pour l'autre. Plus spécialement, les moyens de verrouillage sont réversibles pour une couple de parties creuses n'assurant qu'un simple maintien du tube T.

Selon les exécutions, les moyens d'association comprennent au moins une saillie ménagée sur l'une des parties, apte à coopérer de façon amovible avec au moins un trou borgne ou creux ou rainure complémentaires, ménagé sur l'autre partie de la même section, ou comprennent au moins une pièce amovible de liaison en forme de clip, apte à associer de façon amovible les deux parties de façon indirecte, ou comprennent une pièce amovible de liaison en forme de berceau.

Selon la réalisation en question, les parties creuses sont placées dans le prolongement l'une de l'autre en ménageant entre elles un passage médian débouchant sur la face extérieure du dispositif par un accès, le passage de pincement positif de fermeture comportant au moins un tronçon auquel est adjacent axialement un espace dans lequel débouche le passage et où le tube est d'une part maintenu et d'autre part aplati avec un certain degré.

Dans ce cas, l'accès d'une part, le passage médian et l'espace d'autre part, ont pour fonction, respectivement, de permettre d'une part l'introduction, d'autre part le passage d'un organe de coupe du tube dans sa zone située dans le dit espace.

Selon un second aspect, l'invention propose un ensemble de pincement de fermeture et de sectionnement comprenant un dispositif tel qu'il vient d'être décrit et un organe de coupe, associés fonctionnellement, l'organe de coupe étant apte à être introduit dans le dispositif par l'accès prévu à cet effet et à passer dans le passage médian et l'espace afin de couper le tube dans sa zone située dans l'espace, sans que l'organe de coupe ne sectionne, ne pénètre dans, ou n'interfère frontalement avec le dispositif.

On décrit maintenant plusieurs modes de réalisation de l'invention à l'aide des dessins dans lesquels :
- la figure 1 est une vue en perspective d'une réalisation possible d'un dispositif de pincement de fermeture à l'état ouvert, avec une portion de tube en position, dans laquelle les deux sections du dispositif sont deux pièces distinctes et sont amenées à l'état fermé par un mouvement relatif de coulissement transversal par rapport à l'axe longitudinal du dispositif,
- la figure 2 est une vue en coupe transversale du dispositif de pincement de fermeture à l'état fermé, avec une portion de tube en position,
- la figure 2A est une vue partielle à plus grande échelle de la figure 2,
- la figure 3 est une vue en perspective d'une réalisation possible d'un ensemble de pincement de fermeture et de sectionnement comportant un dispositif de pincement de fermeture du type de celui de la figure 1, dans une réalisation double, à l'état ouvert, avec une portion de tube en position, un organe de coupe étant représenté schématiquement,
- la figure 4 est une vue en coupe axiale, partielle, à plus grande échelle de l'ensemble de pincement de fermeture et de sectionnement et d'une portion de tube en position, juste avant le début du sectionnement du tube,
- la figure 5 est une vue en coupe longitudinale d'une réalisation possible d'un dispositif de pincement de fermeture à l'état fermé, avec une portion de tube en position,
- les figures 6, 7 et 8 sont trois vues en perspective de trois réalisations possibles de moyens de verrouillage d'un ensemble de pincement de fermeture et de sectionnement représenté partiellement,
- la figure 9 est une vue en perspective d'une réalisation possible de l'organe de coupe associé à un ensemble de pincement de fermeture et de sectionnement représenté partiellement,
- la figure 10 est une vue en perspective d'une réalisation possible d'un dispositif de pincement de fermeture à l'état ouvert, dans laquelle les deux sections du dispositif sont deux pièces associées structurellement l'une à l'autre à pivotement autour d'un axe qui, par rapport à l'axe longitudinal du dispositif est déporté sur un côté longitudinal du dispositif, les deux sections étant amenées à l'état fermé par un mouvement relatif de pivotement autour de l'axe de pivotement,
- les figures 11 et 12 sont deux vues en perspective d'une réalisation possible d'un ensemble de pincement de fermeture et de sectionnement comportant un dispositif de pincement de fermeture du type de celui de la figure 10, dans une réalisation double, respectivement désassemblé et assemblé.

Un dispositif 1 de pincement de fermeture permet d'assurer un pincement de fermeture d'un tube T tel que précédemment défini, dans la région souhaitée, raison pour laquelle il est désigné dispositif 1 de pincement de fermeture. Le dispositif 1 de pincement de fermeture, combiné fonctionnellement avec au moins un organe de coupe 2, intégré ou extérieur à lui, forme un ensemble de pincement de fermeture et de sectionnement 1 + 2 qui permet également d'assurer le sectionnement transversal du tube T.

Une fois que le dispositif 1 de pincement de fermeture a été mis en oeuvre pour assurer le pincement positif de fermeture du tube T et également, le cas échéant, son sectionnement transversal, le dispositif 1 de pincement de fermeture a vocation à rester fixé sur le tube T.

Selon une caractéristique, le dispositif 1 de pincement de fermeture est à usage unique.

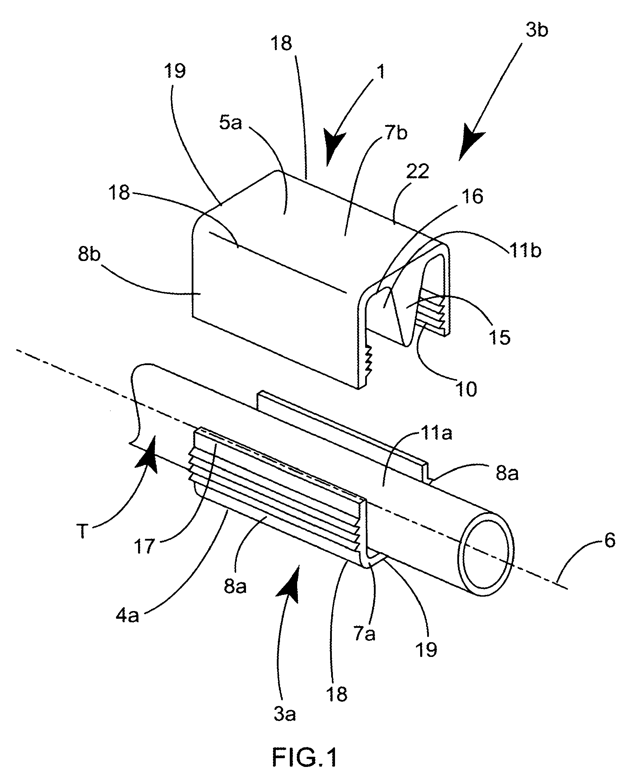

La figure 1 illustre une réalisation possible d'un dispositif 1 de pincement de fermeture comprenant deux sections 3, creuses, profilées, à savoir une première section mâle 3a et une seconde section femelle 3b.

La section mâle 3a a une partie creuse et profilée 4a et la section femelle 3b a une partie creuse et profilée 5a.

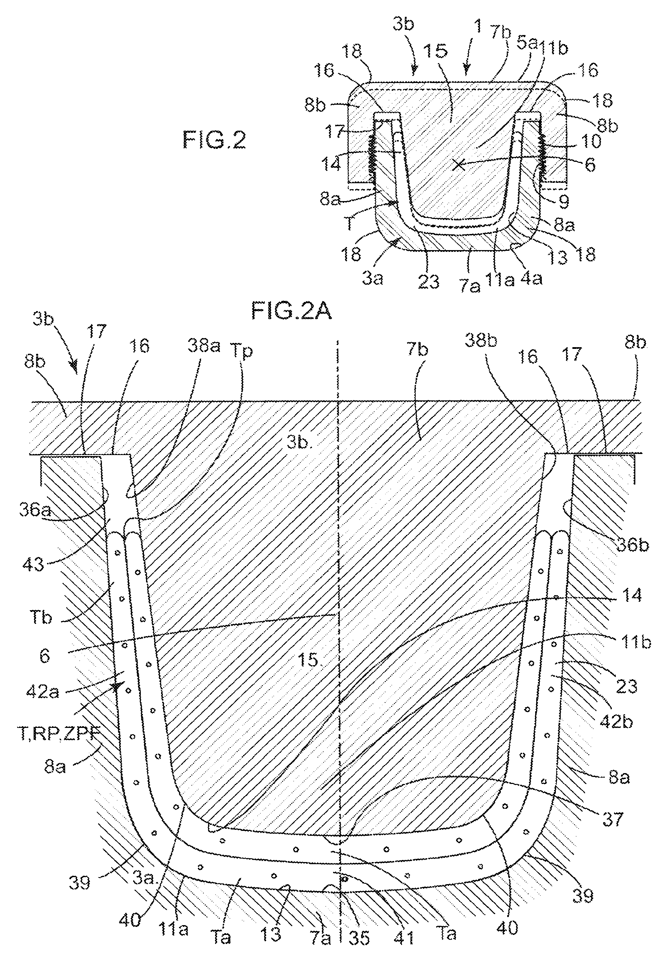

La figure 2 illustre un ensemble de pincement de fermeture et de sectionnement 1 + 2 qui comprend un dispositif 1 de pincement de fermeture du type précédent, mais double, et l'organe de coupe 2.

Dans ce cas, chaque section 3a, 3b comporte deux parties également creuses et profilées, à savoir, respectivement, les deux parties 4a et 4b pour la section mâle 3a et les deux parties 5a et 5b pour la section femelle 3b.

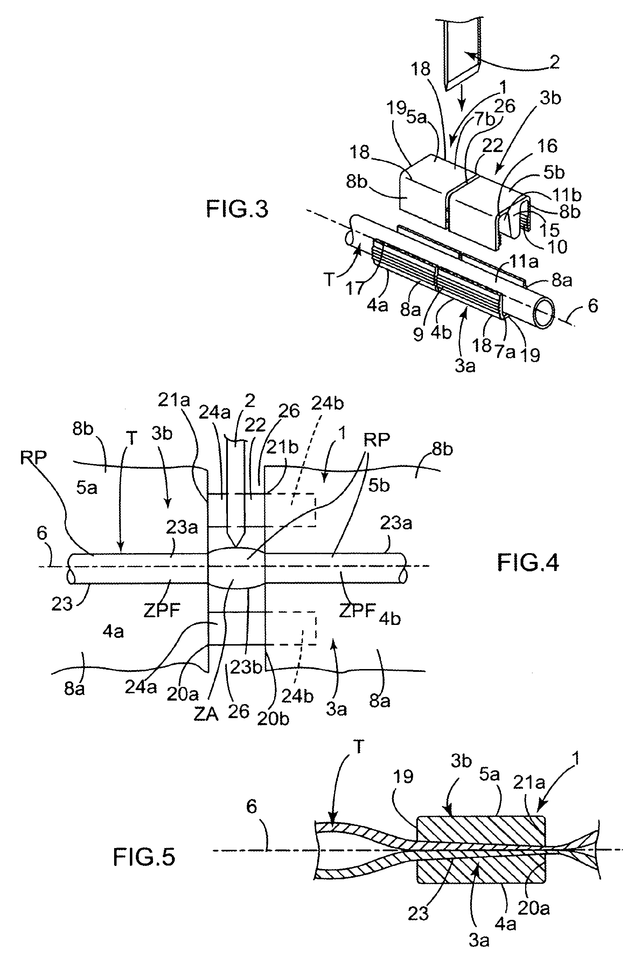

Dans le cas d'un ensemble de pincement de fermeture et de sectionnement 1 + 2, les deux sections 3a et 3b sont aptes, en fonction des phases opératoires, à être structurellement et fonctionnellement associées entre elles par un emboîtement normalement irréversible, pour former un ensemble rigide, les deux parties creuses étant en regard (figure 4), (phase de pincement, phase de sectionnement) ou à être dissociées (figure 3) (phase de mise en place).

Les deux parties 4a et 4b ou 5a et 5b d'une même section 3a ou 3b sont aptes, selon les phases opératoires, à être structurellement et fonctionnellement associées entre elles en étant placées dans le prolongement l'une de l'autre (figure 3) (phase de pincement, phase de sectionnement), ou à être dissociées (figures 5, 6 et 7) (phase de dissociation).

Lorsque les deux sections 3a et 3b sont structurellement et fonctionnellement associées entre elles par emboîtement, leurs parties en regard respectives 4a et 5a d'une part, 4b et 5b d'autre part sont également structurellement et fonctionnellement associées entre elles par un emboîtement qui, dans les conditions normales d'utilisation est irréversible.

Les sections sont référencées de façon générique 3 et les parties 4 et 5.

Le dispositif 1 de pincement de fermeture comprend donc les quatre pièces distinctes 4a, 4b, 5a et 5b, compactes, profilées, rigides et typiquement réalisées en matière plastique telle qu'un polymère choisi pour satisfaire, le cas échéant, les autres contraintes auxquelles le dispositif 1 de pincement de fermeture doit faire face, par exemple, résister à un traitement en autoclave ou par rayonnement y.

Dans une réalisation, les parties 4a et 4b de la section 3a sont, sinon strictement identiques, du moins analogues entre elles. De même en ce qui concerne les parties 5a et 5b de la section 3b. Dans ce cas, le dispositif 1 de pincement de fermeture assure une fonction de fermeture du tube T sur lui-même dans deux zones de pincement positif de fermeture.

Mais, dans une autre réalisation, les deux parties creuses 4a, 4b et 5a, 5b ne sont pas analogues, mais différentes, de sorte que, par exemple, le dispositif 1 de pincement de fermeture n'assure une fonction de fermeture du tube T sur lui-même que dans une seule zone de pincement positif de fermeture. Dans son autre zone, le tube T est alors simplement maintenu, ce qui facilite son sectionnement transversal. Le cas échéant, ce maintien est amovible, n'existant que tant que nécessaire pour le sectionnement.

Le dispositif 1 de pincement de fermeture présente un axe longitudinal 6 qui est également l'axe longitudinal du tube T lorsque celui-ci est placé dans le dispositif 1 de pincement de fermeture. C'est en relation avec cet axe 6 qu'est qualifié de « axial » toute direction ou plan s'étendant parallèlement à l'axe 6 et de « transversal » toute direction ou plan s'étendant orthogonalement à l'axe 6.

Les deux sections 3a et 3b sont profilées et s'étendent le long de l'axe 6.

Dans la réalisation représentée, les deux sections 3a et 3b sont monobloc et de même longueur axiale, de manière à pouvoir être emboîtées en étant ajustées, en regard l'une de l'autre.

Chacune des deux sections 3a et 3b, respectivement chacune de ses deux parties 4a, 4b, et 5a, 5b, a un contour extérieur ayant en section droite transversale une forme générale de U ou de pseudo U. Ce contour comporte, et est limité par, plusieurs tronçons, à savoir une âme et, de part et d'autre de celle-ci, deux rives latérales, l'âme et les deux rives s'étendant par ailleurs axialement.

La première section mâle 3a comporte, et est limitée extérieurement par, une première âme 7a et deux premières rives 8a. La seconde section femelle 3b comporte, et est limitée extérieurement par, une seconde âme 7b et deux secondes rives 8b.

L'écartement entre les deux faces extérieures 9 opposées des deux premières rives 8a est en correspondance avec l'écartement entre les deux faces intérieures 10 en regard des deux secondes rives 8b. Cette disposition permet, à l'état fermé du dispositif 1 de pincement de fermeture, l'emboîtement avec ajustement des deux sections 3a et 3b, respectivement de leurs parties constitutives, la couple de faces extérieures 9 étant alors en contact et en appui sur la couple de faces intérieures 10.

Dans la réalisation représentée, la première section mâle 3a définit une première empreinte femelle 11a et la seconde section femelle 3b définit une seconde empreinte mâle 11 b.

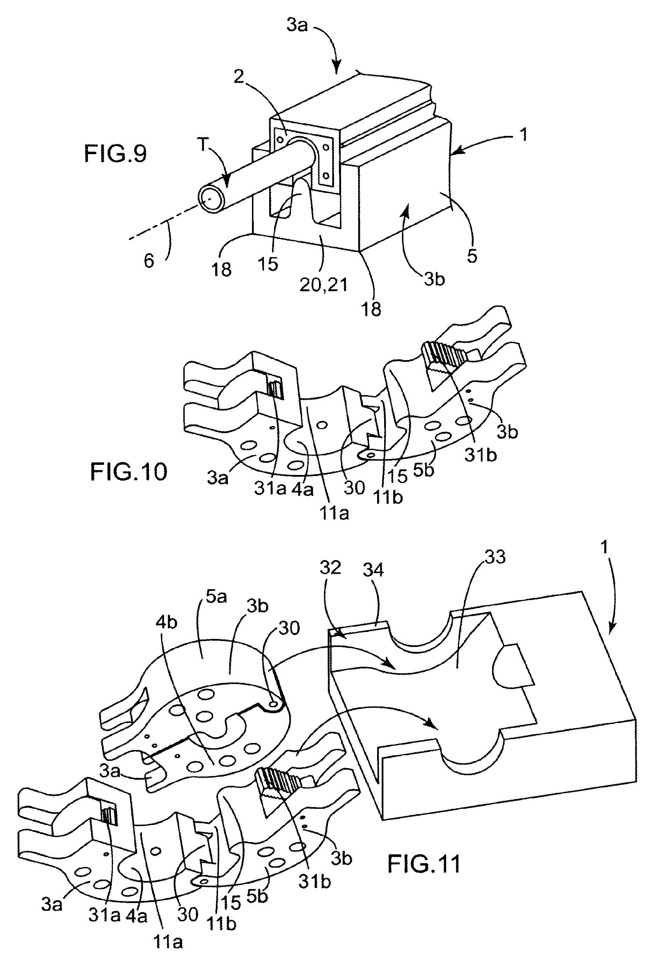

Selon une autre réalisation, la disposition est inverse, la première section mâle 3a définissant une première empreinte mâle et la seconde section femelle 3b définissant une seconde empreinte femelle (figure 9).

La première empreinte femelle 11a est définie par une face 13 profilée, s'étendant le long de l'axe 6, ayant en section transversale un contour de l'intrados d'un U et s'étendant parallèlement à l'axe 6. La face 13 est constituée de la face intérieure 35 de la première âme 7a, disposée de façon médiane, et des deux faces intérieures 36a et 36b des deux premières rives 8a, disposées latéralement de part et d'autre de la face intérieure 35. Ainsi, l'empreinte femelle 11 a comporte une âme 35 et deux ailes 36a et 36b.

La seconde empreinte mâle 11 b est définie par une face 14 profilée, s'étendant le long de l'axe 6, ayant en section transversale un contour de l'extrados d'un U et s'étendant parallèlement à l'axe 6. La face 14 est constituée de la face extérieure 37 de la partie d'extrémité d'une saillie 15, disposée de façon médiane, et des deux faces extérieures 38a et 38b du corps de la saillie 15, placées de part et d'autre de la face extérieure 37. Ainsi, l'empreinte mâle 11 b comporte une âme 37 et deux ailes 38a et 38b.

La saillie 15 est attenante à la seconde âme 7b et s'étend à égale distance entre les deux secondes rives 8b en étant dirigée dans le même sens qu'elles.

La face 14 est reliée aux deux faces intérieures 10 des deux rives 8b par deux petites faces sensiblement coplanaires 16. Ces deux petites faces 16 sont susceptibles, le cas échéant, lorsque l'emboîtement des deux sections 3a et 3b est le plus grand, d'être en contact et en appui sur les deux chants libres 17 des deux rives 8a, à l'état fermé du dispositif 1 de pincement de fermeture.

Bien entendu, pour pouvoir réaliser l'emboîtement requis, les deux faces 13 et 14 sont disposées tête-bêche l'une par rapport à l'autre. Si l'une est disposée en U, l'autre est disposée en ∩ (U inversé).

Les faces 13 et 14 sont conjuguées de manière à pouvoir coopérer entre elles et assurer le pincement du tube T sur lui-même jusqu'à sa fermeture totale, les âmes 35 et 36 étant conjuguées entre elles, de même que d'une part les ailes 36a et 38a, d'autre part les ailes 36b et 38b. Comme les empreintes 11 a, 11 b, le pincement s'étend le long de l'axe 6, c'est-à-dire qu'il n'est pas ponctuel, mais linéaire sur une certaine longueur.

Les faces 13 et 14 sont globalement lisses. Dans la réalisation représentée, les faces 13 et 14 ont des formes simples et non pas des formes tourmentées.

Dans la réalisation représentée, les âmes 35 et 37 ont une forme globalement aplanie avec une légère incurvation, respectivement concave et convexe. Les ailes 36a, 36b, 38a, 38b ont une forme globalement aplanie.

Les deux ailes 36a et 36b de la couple d'ailes 36a, 36b de l'empreinte femelle 11a sont légèrement divergentes à partir de l'âme 35, de manière que l'ouverture de l'empreinte femelle 11 b soit légèrement évasée. Ces ailes 36a et 36b sont reliées à l'âme 35 par deux parties arrondies 39 de rayon de courbure suffisamment important.

Les deux ailes 38a et 38b de la couple d'ailes 38a, 38b de l'empreinte mâle 11 b sont légèrement convergentes à partir de la seconde âme 7b qui constitue la base de la saillie 15. Ces ailes 38a et 38b sont reliées à l'âme 37 par deux parties arrondies 40 de rayon de courbure suffisamment important.

Les faces 13 et 14 ont des contours globalement analogues, comme il est décrit. Elles sont destinées à être disposées globalement dans la même direction. Elles sont écartées transversalement l'une de l'autre, par suite de la présence entre elles du tube T positivement pincé et fermé, à raison de la géométrie qu'elles présentent. Elles sont maintenues ainsi grâce à des moyens de verrouillage sur lesquels on reviendra.

Le dispositif 1 de pincement de fermeture et ses parties constitutives (sections 3 et parties 4 et 5), peuvent se trouver dans l'un des deux états suivants : état ouvert et état fermé.

A l'état ouvert (figures 1 et 3), les deux sections 3a et 3b sont écartées et dissociées l'une de l'autre. Les deux sections 3a et 3b peuvent alors être manipulées chacune séparément. Dans cet état ouvert, les deux sections 3a et 3b laissent un libre accès transversal aux empreintes 11a et 11b, chacune d'elle n'étant pas recouverte par la section à laquelle appartient l'autre empreinte.

A l'état ouvert, le tube T peut, dans toute région longitudinale souhaitée pour être une région de pincement RP, être introduit dans, ou si nécessaire enlevé de, l'empreinte femelle 11a formant berceau, sans que sa forme ne soit affectée, en particulier qu'il soit pincé ou aplati, la circulation, le passage de sectionnement, la communication du fluide dans le tube T n'étant pas bloqué, empêché, ou entravé. Le berceau de l'empreinte femelle 11a est bien entendu adapté à la forme et à la dimension transversale du tube T.

Ainsi, l'état ouvert permet la phase de mise en place du tube T dans le dispositif 1 de pincement de fermeture, éventuellement son enlèvement (figures 1 et 3).

A l'état fermé (figure 2), les deux sections 3a et 3b sont proches l'une de l'autre et emboîtées, les deux rives 8a de la première section mâle 3a venant se loger avec contact de maintien, dans les deux rives 8b de la seconde section femelle 3b.

A l'état fermé, les deux faces extérieures 9 des premières rives 8a sont en contact et en appui sur les deux faces intérieures 10 des secondes rives 8b, et, le cas échéant - comme indiqué précédemment -, les deux petites faces 16 sont en contact et en appui sur les deux chants libres 17 des secondes rives 8b.

A l'état fermé, le dispositif 1 de pincement de fermeture présente une forme générale extérieure qui est sensiblement celle d'un parallélépipède à section transversale au moins sensiblement carrée, allongé le long de l'axe 6, et dont les quatre arêtes 18 s'étendant axialement, sont arrondies. Ce parallélépipède comporte deux extrémités libres ou faces transversales d'extrémité et distales 19 (figure 5).

On se réfère au cas où le dispositif 1 de pincement de fermeture fait partie d'un ensemble de pincement de fermeture et de sectionnement 1 + 2, chaque section 3a, 3b comportant deux parties 4a et 4b, 5a et 5b.

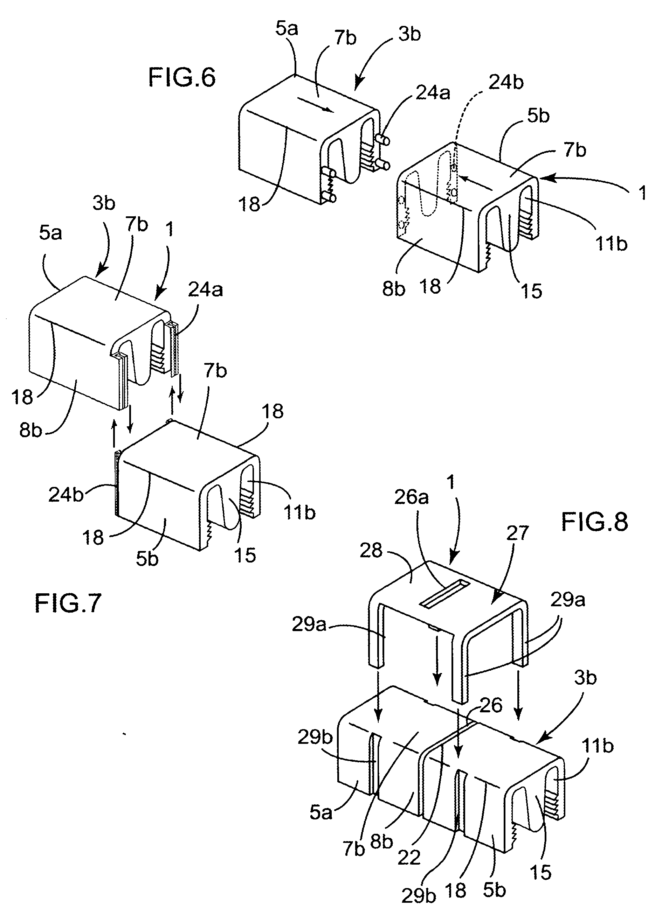

Chacune des parties 4a, 4b, 5a, 5b est terminée du côté extérieur par une extrémité libre ou face transversale d'extrémité distale 19 et, du côté intérieur, par une face transversale d'extrémité et proximale, à savoir, respectivement, 20a, 20b, 21 a, 21 b (figures 5 et 6). Ces faces transversale d'extrémité et proximales sont substantiellement planes.

A l'état fermé, les deux parties 4a et 4b, 5a et 5b de chacune des deux sections 3a et 3b ont leurs deux faces transversales d'extrémité et proximales 20a et 20b, 21 a et 21 b situées en regard l'une de l'autre et à proximité immédiate l'une de l'autre mais néanmoins écartées l'une de l'autre, le long de l'axe longitudinal 6, de manière à ménager un passage transversal médian de sectionnement 22. Les deux faces transversales d'extrémité et proximales 20a et 21a des parties 4a et 5a, comme les deux faces transversales d'extrémité et proximales 20b et 21 b des parties 4b et 5b sont coplanaires ou sensiblement coplanaires.

A l'état fermé, les deux empreintes 11a et 11b n'ont plus d'accès transversal libre comme dans l'état ouvert, mais elles ménagent entre elles un passage de pincement positif de fermeture 23, d'axe 6, formant une sorte de canal creux, limité par les deux faces 13 et 14 des empreintes 11 a et 11 b, et la partie des petites faces 16 attenantes à la saillie 15.

Dans cette réalisation de l'ensemble de pincement de fermeture et de sectionnement 1 + 2, le passage de pincement positif de fermeture 23 est en deux tronçons de pincement positif de fermeture 23a placés dans la ligne de prolongement l'un de l'autre. Ces deux tronçons 23a débouchent tout d'abord des deux faces transversales d'extrémité et distales 19, vers l'extérieur du dispositif 1 de pincement de fermeture. Ces deux tronçons 23a débouchent ensuite des deux couples de faces transversales d'extrémité et proximales 20a, 21 a et 20b, 21b.

Entre les deux couples de faces transversales d'extrémité et proximales 20a, 21 a et 20b, 21b, le passage de pincement positif de fermeture 23 est absent pour ne pas exister, les couples de parties 4a, 5a et 4b, 5b étant écartés l'une de l'autre en direction axiale, sur une faible distance, comme indiqué précédemment.

Les deux tronçons de pincement positif de fermeture 23a sont donc placés dans le prolongement l'un de l'autre, mais avec un petit écartement entre eux 23b.

En se référant à la figure 4, on appelle par convention « zone de pincement positif de fermeture » ZPF du tube T, le tronçon longitudinal du tube T se trouvant au droit des parties 4a et 5a, 4b et 5b du dispositif 1 de pincement de fermeture, dans les deux tronçons de pincement positif de fermeture 23a du passage 23. On appelle « zone d'aplatissement » ZA du tube T, le tronçon longitudinal du tube T se trouvant entre les parties 4a et 5a, 4b et 5b du dispositif 1 de pincement de fermeture, dans l'écartement 23b entre les deux tronçons 23a du passage 23. La région de pincement RP du tube T déjà mentionnée est constituée par le tronçon longitudinal du tube T comprenant les deux zones de pincement positif de fermeture ZPF et la zone d'aplatissement ZA placé entre elles.

Dans cette réalisation, les parties 4a et 4b d'une part, 5a et 5b d'autre part sont analogues, de sorte que le dispositif 1 de pincement de fermeture assure une fonction de fermeture du tube T sur lui-même dans les deux zones de pincement positif de fermeture ZPF, s'étendant sur une certaine longueur axiale, séparées l'une de l'autre par la zone d'aplatissement ZA. Dans cette zone, le tube T est maintenu, sans qu'une fermeture totale soit positivement assurée. Ce maintien du tube T facilite son sectionnement, le dispositif 1 de pincement de fermeture faisant ici partie d'un ensemble de pincement de fermeture et de sectionnement 1 +2.

Dans une autre réalisation d'un tel ensemble, on peut prévoir que le dispositif 1 de pincement de fermeture assure une fonction de fermeture du tube T sur lui-même dans une seule zone de pincement positif de fermeture ZPF. Ce qui, dans la réalisation précédente était l'autre zone de pincement positif de fermeture ZPF peut n'assurer que le maintien positif, sans fermeture totale, la zone d'aplatissement ZA jouant alors le même rôle que dans la réalisation précédente. Dans ce cas, il n'est pas indispensable que les parties creuses assurant ce simple maintien positif comportent des empreintes telles que celles précédemment décrites.

Le passage de pincement 23 a, en section droite transversale, une forme de U, par suite de la forme correspondante des empreintes 11 a, 11 b. Ce passage 23 comporte par conséquent une âme 41 et deux ailes 42a et 42b, correspondant, respectivement, aux âmes 35 et 37 et aux ailes 36a, 38a et 36b, 38b.

Le tube T disposé dans le passage de pincement 23 du dispositif 1 de pincement de fermeture à l'état fermé, contraint par le passage 23 profilé, est conformé en étant, d'une part aplati sur lui-même et ainsi fermé, d'autre part conformé en U, avec une âme Ta et deux ailes Tb, terminées par deux plis d'extrémité Tp.

La largeur (dans un plan transversal) de l'ouverture que forme le passage de pincement 23 - âme 41 et ailes 42a et 42b - est définie par l'écartement transversal entre la face intérieure 13 et la face 14, à savoir l'écartement transversal entre les âmes 35 et 37, d'une part et les ailes en regard des couples d'ailes 36a et 38a, 36b et 38b.

A l'état fermé, les deux ailes 36a et 38a, 36b et 38b de chaque couple d'ailes en regard de l'empreinte mâle 11 a et de l'empreinte femelle 11 b vont en se rapprochant l'une de l'autre vers leurs extrémités 43 opposées aux âmes 35 et 37 des empreintes, au moins dans la zone de ces extrémités 43. Les sections 3a et 3b sont agencées en conséquence.

Dans la réalisation représentée, les deux ailes 36a et 38a, 36b et 38b vont en se rapprochant l'une de l'autre de façon plus ou moins continue depuis les âmes 35 et 37 jusqu'aux extrémités 43 opposées.

Par suite, chacune des deux ailes 42a et 42b du passage 23 a, entre les faces 36a, 38a et 36b, 38b des deux empreintes, une ouverture dont la largeur va en diminuant vers son extrémité opposée à l'âme 41 du passage 23, et notamment va en diminuant de façon plus ou moins continue à partir de l'âme 41.

La plus grande largeur du passage 23 est légèrement plus petite que le double de l'épaisseur de la paroi du tube T, en fonction de la compressibilité du tube T, de manière que le tube T une fois placé dans le dispositif 1 de pincement de fermeture à l'état fermé soit comprimé sur lui-même et que le dispositif assure une fonction de fermeture du tube T sur lui-même.

Par suite du fait que la largeur du passage 23 va en diminuant, vers son extrémité opposée à l'âme 41, le tube T est davantage comprimé dans la zone de ses deux plis Tp. Cette disposition permet d'éviter que ne subsiste dans la zone des plis une petite ouverture du tube sur lui-même, une telle ouverture ayant pour effet de permettre à une ou plusieurs gouttes du fluide du tube T de s'écouler de lui lors du pincement de fermeture et le sectionnement du tube T.

Ainsi, le dispositif 1 de pincement de fermeture remplit une fonction de fermeture du tube T sur lui-même dans la région de pincement empêchant tout passage du fluide dans le tube T.

Une fois le tube T, dans la phase de mise en place, positionné dans l'empreinte femelle 11a, alors que le dispositif 1 de pincement de fermeture est à l'état ouvert, le dispositif 1 de pincement de fermeture est amené à l'état fermé, le tube T est emprisonné dans le passage 23 dans la phase de pincement.

Dans la phase de pincement, le tube T est pincé positivement et fermé dans ses deux zones de pincement positif de fermeture ZPF par les faces 13 et 14 et la coopération des empreintes 11 a et 11 b. Entre les deux zones de pincement positif de fermeture ZPF, le tube T n'est pas positivement pincé et fermé, mais il est aplati plus ou moins fortement avec un certain degré, ce qui justifie que ce tronçon de tube T soit qualifié de zone d'aplatissement ZA. Par suite du pincement positif de fermeture, le tube T est fermé sur lui-même dans la région de pincement RP, ce qui empêche la circulation, le passage, la communication du fluide de part et d'autre de cette région.

Avec le au moins un organe de coupe 2, le tube T peut également, dans une phase de sectionnement ultérieure à la phase de pincement, être sectionné transversalement dans le passage de sectionnement 22, dans la zone d'aplatissement ZA et donc dans la région de pincement RP. Le sectionnement intervenant après la fermeture, il n'est en aucune manière perturbé par le fluide se trouvant dans le tube T. La fermeture étant totale, le risque que s'écoulent une ou plusieurs gouttes du fluide du tube T est évité.

Les formes et dimensions du dispositif 1 de pincement de fermeture sont adaptées à la nature, à la forme et aux dimensions du tube T, de manière que le pincement de fermeture souhaité en ce qui concerne le tube T soit correctement réalisé.

Dans la réalisation de la figure 3, l'empreinte femelle 11 a et l'empreinte mâle 11b ont une même forme, une même disposition et de mêmes dimensions, constantes et uniformes, d'une extrémité libre et distale 19 à l'autre extrémité libre et distale 19, via les extrémités proximales.

Afin de pouvoir être placées et maintenues dans le prolongement l'une de l'autre (figure 3), les deux parties 4a et 4b ou 5a et 5b d'une même section 3a ou 3b sont structurellement et fonctionnellement associées entre elles, de façon amovible, par des moyens d'association.

Plus spécialement, les deux parties 4a et 4b ou 5a et 5b sont associés par leurs faces d'extrémité et proximales, respectivement 20a et 20b d'une part, 21 a et 21 b d'autre part (écartées le long de l'axe 6 pour former le passage de sectionnement 22), grâce à des moyens d'association 24a et 24b appartenant aux parties 4a et 4b d'une part, 5a et 5b d'autre part.

Dans la réalisation des figures 3 et 6, les moyens d'association 24 comprennent plusieurs (ici quatre mais pouvant être d'un nombre différent) saillies ou ergots formant tétons 24a ménagés sur la face d'extrémité et proximale 20a, 20b, 21 a, 21 b de l'une des deux parties 4a, 4b, 5a, 5b, aptes à coopérer de façon amovible avec un nombre égal de trous borgnes ou creux formant cavités 24b, complémentaires, ménagés sur la face d'extrémité et proximale 20b, 20a, 21 b, 21 a de l'autre des deux parties 4b, 4a, 5b, 5a.

Ces saillies 24a et ces trous 24b s'étendent parallèlement à l'axe 6, et donc à la direction d'extension du tube T lorsqu'il est positionné dans le dispositif 1 de pincement de fermeture. Leur coopération est réalisée par un coulissement axial parallèlement à l'axe 6. Leurs dimensions sont telles que les saillies 24a viennent se loger avec le jeu minimal dans les trous 24b.

Pour que le maintien des parties 4a et 4b d'une part, 5a et 5b d'autre part soit assuré de façon efficace, les saillies 24a et les trous 24b sont disposées sur les paires de rives 8a d'une part, 8b d'autre part, de part et d'autre des empreintes 11 a et 11 b et du passage de sectionnement 22.

Afin de ne pas obstruer l'accès et l'ouverture du passage de sectionnement 22, il est prévu qu'aucune saillie et aucun trou tel que 24a, 24b ne se trouve au droit des âmes 7a et 7b.

Selon d'autres réalisations, le nombre de saillies 24a et de trous 24b est différent, ou il est prévu sur une même face d'extrémité et proximale donnée 20a, 20b, 21 a, 21 b, non pas un ou plusieurs saillies 24a ou trous 24b, mais une combinaison de saillies 24a et de trous 24b.

Le passage transversal de sectionnement 22 est réalisé par la présence de moyens d'écartement prévus à cet effet. Ces moyens d'écartement sont constitués par des butées ménagés soit sur les faces d'extrémité et proximales 20a, 20b, 21 a, 21 b soit sur les moyens d'association 24a, 24b, par exemple, les saillies 24a sont bloquées en fin de course dans les trous 24b.

Dans la réalisation du dispositif 1 de pincement de fermeture représenté sur la figure 1, les deux sections 3a et 3b comprennent des moyens 25a et 25b de verrouillage réciproque des deux sections 3a et 3b et des parties 4a et 5a à l'état fermé. Ces moyens de verrouillage 25a et 25b sont irréversibles, les deux sections 3a et 3b une fois amenées à l'état fermé étant maintenues dans cet état par les moyens de verrouillage 25a et 25b, sans pouvoir être dissociées.

Ainsi, une fois que le tube T logé dans le passage 23 a été pincé et fermé, le dispositif 1 de pincement de fermeture ayant été amené à l'état fermé, le tube T est maintenu ainsi pincé et fermé et il reste dans cet état, le dispositif 1 de pincement de fermeture restant associé au tube T par suite de l'irréversibilité des moyens de verrouillage 25a et 25b.

Dans la réalisation de l'ensemble de pincement de fermeture et de sectionnement 1 + 2 représenté sur la figure 3, dans laquelle le pincement positif de fermeture intervient dans deux zones de pincement positif de fermeture ZPF, les deux sections 3a et 3b comprennent des moyens 25a et 25b de verrouillage réciproque des deux sections 3a et 3b et des deux couples de parties 4a, 5a et 4b, 5b à l'état fermé.

Dans une variante de cette réalisation, l'irréversibilité des moyens de verrouillage 25a et 25b est prévue pour l'une seulement des deux couples de parties 4a, 5a et 4b, 5b. Cette variante est utile lorsque l'on souhaite qu'une extrémité de sectionnement soit fermée sans nécessité que l'autre extrémité de sectionnement le soit, les parties correspondantes des sections 3a et 3b pouvant alors être enlevées.

Dans une autre réalisation d'un ensemble de pincement de fermeture et de sectionnement 1 + 2 où le dispositif 1 de pincement de fermeture assure une fonction de fermeture du tube T sur lui-même dans une seule zone de pincement positif de fermeture ZPF, l'autre zone n'assurant qu'un maintien sans fermeture totale, on peut également prévoir que l'irréversibilité des moyens de verrouillage 25a et 25b n'est prévue que pour les parties des sections 3a et 3b assurant le pincement positif de fermeture, mais n'est pas prévue pour les parties n'assurant qu'un maintien sans fermeture totale.

Dans la réalisation des figures 3 et 6, les moyens de verrouillage réciproque 25a et 25b sont deux ensembles comprenant une série de dents d'accrochage irréversible profilées (ou des crans), orientées selon deux directions opposées, s'étendant axialement, ménagées respectivement sur les deux faces extérieures 9 des premières rives 8a et les deux faces intérieures 10 des secondes rives 8b dans leur zone d'emboîtement.

A la place des dents, on peut prévoir des crans ou analogue.

Dans la réalisation des figures 1 à 3, les moyens de verrouillage réciproque 25a et 25b sont amenés à coopérer l'un avec l'autre consécutivement à un mouvement de coulissement de rapprochement des deux sections 3a et 3b selon une direction transversale, conduisant à l'emboîtement à force des deux sections 3a et 3b, les dents 25a et 25b venant en prise mutuellement de façon irréversible.

Le passage de sectionnement 22 débouche sur la face extérieure du dispositif 1 de pincement de fermeture par au moins un accès, en l'espèce une fente d'accès 26, apte à permettre l'introduction dans le passage de sectionnement 22, et l'enlèvement depuis le passage de sectionnement 22, de l'organe de coupe 2, par un mouvement de coulissement transversal ou d'un organe de commande du mouvement de l'organe de coupe 2. D'autre part, le passage de pincement 23 débouche dans le passage de sectionnement 22.

Dans la réalisation des figures 3 et 6, le passage de sectionnement 22 est traversant de la fente 26 à une fente analogue diamétralement opposée située également à la périphérie du dispositif 1 de pincement de fermeture. Dans une autre réalisation non représentée, le passage de sectionnement 22 est non traversant ou borgne, ne débouchant pas à la périphérie diamétralement opposée du dispositif 1 de pincement de fermeture, auquel cas il n'y a qu'une seule fente 26.

Lorsqu'il est prévu deux fentes diamétralement opposées, on peut, selon les réalisations prévoir un seul organe de coupe 2 (ou organe de commande du mouvement de l'organe de coupe 2) ou deux organes de coupe 2 opposés (ou organes de commande), chacun étant introduit par une fente dédiée.

Le passage de sectionnement 22 débouche également dans, et interfère avec, l'espace ou écartement 23a situé entre les deux tronçons de pincement positif de fermeture 23a.

L'accès 26, disposé transversalement, comme le passage de sectionnement 22, comporte un grand côté ou longueur et un petit côté ou largeur. Le grand côté s'étend transversalement au droit de l'ouverture du berceau de la face 13 de la première empreinte 11a, et a une longueur au moins légèrement plus grande que cette ouverture. Avec cette disposition constructive, on est assuré que l'organe de coupe 2 vient bien interférer avec la totalité d'une section transversale du tube T maintenu pincé et fermé dans les deux tronçons de pincement positif de fermeture 23a et traversant l'espace ou écartement 23b situé entre eux. Et, ainsi, l'organe de coupe 2 peut sectionner transversalement le tube T dans l'espace ou écartement 23b.

A cet effet, les moyens d'association 24a, 24b des sections 3 sont espacés de la face 13, de manière à permettre le passage de l'organe de coupe 2 entre les moyens d'association 24a, 24b, sans les couper ou même les entamer.

Le petit côté de l'accès 26 est disposé axialement. Sa longueur, faible, est adaptée à l'épaisseur de l'organe de coupe 2 (ou de l'organe de commande du mouvement de l'organe de coupe 2), de manière à permettre le passage et le guidage de l'organe de coupe 2 (ou de l'organe de commande) dans le passage de sectionnement 22, sans que l'organe de coupe 2 ne coupe ou même n'entame le dispositif 1 de pincement de fermeture lui-même.

Le passage de sectionnement 22 et de l'accès 26 sont dimensionnés en fonction des dimensions du dispositif 1 de pincement de fermeture, de la nature de la forme et des dimensions du tube T, ainsi que des dimensions de l'organe de coupe 2 ou de l'organe de commande de l'organe de l'organe de coupe 2, de manière à assurer un sectionnement efficace.

Bien entendu, il est prévu les jeux nécessaires au mouvement de l'organe de coupe.

Le dispositif 1 de pincement de fermeture qui vient d'être décrit est mis en oeuvre comme il est maintenant décrit, dans le but de pincer un tube T et, le cas échéant et alors en combinaison avec le au moins un organe de coupe 2, dans le cadre d'un ensemble de pincement de fermeture et de sectionnement 1 + 2, dans le but de couper transversalement le tube T.

On dispose d'un dispositif 1 de pincement de fermeture qui est, ou est amené, à l'état ouvert et dont chaque section 3a et 3b comporte ses deux parties 4a et 4b, et 5a et 5b associées grâce aux moyens d'association 24a et 24b.

On dispose du tube T, qui, dans cette situation est continu et normalement conformé pour laisser passer le fluide.

On positionne le tube T, dans la région de pincement RP souhaitée de celui-ci, dans l'empreinte femelle 11a de la première section mâle 3a (phase de mise en place). Par exemple, la première section mâle 3a est disposée pour que sa face 13 soit tournée frontalement vers l'opérateur ou soit tournée pour être à la vue de l'opérateur. Dans la réalisation de la figure 1, la première section mâle 3a est disposée sensiblement avec l'axe 6 horizontal, sa face 13 tournée vers le haut.

On amène la seconde section femelle 3b en regard de la première section mâle 3a, les faces 13 et 14 étant tournées l'une vers l'autre. Dans la réalisation de la figure 3, la seconde section femelle 3b est disposée au dessus de la première section mâle 3a, sensiblement avec l'axe 6 horizontal, sa face 14 tournée vers le bas à l'aplomb de la face 13.

Par un mouvement relatif de rapprochement des première et seconde sections 3a et 3b, et des parties 4a et 4b d'une part, 5a et 5b d'autre part, en l'espèce par un mouvement relatif de coulissement transversal, on fait passer le dispositif 1 de pincement de fermeture (ses sections 3 et parties 4 et 5) de l'état ouvert antérieur à l'état fermé ultérieur. Dans la réalisation de la figure 3, ce mouvement relatif de coulissement transversal est un mouvement vertical.

Ce mouvement de fermeture est poursuivi jusqu'en fin de course, alors que le tube T est positivement pincé jusqu'à être fermé dans ses deux zones de pincement positif de fermeture ZPF par les faces 13 et 14 qui le déforment et le contraignent ainsi, étant donné la relation géométrique existant entre le tube T et les deux tronçons de pincement positif de fermeture 23a du passage 23 (phase de pincement).

Ce mouvement de fermeture est rendu aisé par suite des moyens de verrouillage 25a, 25b, irréversible, c'est-à-dire anti-retour.

En amenant le dispositif 1 de pincement de fermeture à l'état fermé, on fait simultanément coopérer les moyens de verrouillage réciproque 25a et 25b, pour maintenir fermées les deux parties 4a et 5a d'une part, 4b et 5b d'autre part, de chaque paire de parties des première et seconde sections 3a et 3b, dans la position où le tube T est pincé et fermé.

On maintient le tube T ainsi pincé et fermé entre les deux sections 3a et 3b du dispositif 1 de pincement de fermeture.

Dans une réalisation, on peut se contenter d'en rester là, le dispositif 1 de pincement de fermeture fermé restant fixé au tube T. Dans ce cas, le tube T reste d'un seul tenant et n'est pas sectionné.

Dans une autre réalisation, on souhaite également sectionner le tube T dans la région de pincement RP afin d'obtenir un tube T sectionné en deux longueurs, chacune d'elle pincée et fermée à sa partie extrême sectionnée ou, comme indiqué plus haut, l'une seulement est pincée et fermée, l'autre étant seulement maintenue.

Dans ce cas, on dispose également d'au moins un organe de coupe 2.

Alors que le dispositif 1 de pincement de fermeture est à l'état fermé autour du tube T, dans une phase de sectionnement, on coupe le tube T après que l'on a réalisé son pincement et sa fermeture, dans la zone d'aplatissement ZA dans laquelle le tube n'est pas positivement pincé, mais néanmoins aplati. Selon les réalisations, on sectionne le tube T immédiatement après ou un certain temps après que l'on a réalisé son pincement positif de fermeture.

A cet effet, on amène l'organe de coupe 2 en regard de l'accès 26 du dispositif 1 de pincement de fermeture.

Puis, par un mouvement relatif de rapprochement de l'organe de coupe 2 et du dispositif 1 de pincement de fermeture, en l'espèce un mouvement de coulissement transversal de l'organe de coupe 2 dans son propre plan, on introduit l'organe de coupe 2 par son extrémité dans l'accès 26 et on poursuit le mouvement jusqu'à ce que l'organe de coupe 2 pénètre davantage dans le passage de sectionnement 22, puis dans l'espace ou écartement 23b où il interfère avec le tube T dans sa zone d'aplatissement ZA entre les paires de faces proximales 20a, 21 a et 20b, 21 b. C'est ainsi que l'on coupe ou sectionne le tube T transversalement.

Dans ce mouvement, l'organe de coupe 2 est, le cas échéant, guidé par les paires de faces proximales 20a, 21a et 20b, 21b, et le cas échéant également, par les organes d'association 24a, 24b.

Ainsi, on sectionne le tube T, sans que l'organe de coupe 2 ne sectionne, ne pénètre dans, ou n'interfère frontalement avec le dispositif 1 de pincement de fermeture. Par suite, on évite l'apparition de copeaux ou déchets gênants provenant du dispositif 1 de pincement de fermeture.

Selon les réalisations, le mouvement de coulissement de l'organe de coupe 2 est poursuivi de part en part du dispositif 1 de pincement de fermeture si le passage de sectionnement 22 est traversant ou est arrêté à la position adéquate si le passage de sectionnement 22 est non traversant, borgne. Selon les réalisations, la coupe est faite au moyen d'un seul organe de coupe 2 ou de deux organes de coupe 2 opposés.

Selon les réalisations, on fait passer l'organe de coupe 2 dans le passage de sectionnement 22 sans contact avec le dispositif 1 de pincement de fermeture (figure 4) ou au contraire avec un contact surfacique glissant avec le dispositif 1 de pincement de fermeture ((figure 9).

Une fois le tube T sectionné dans sa zone d'aplatissement ZA, l'organe de coupe 2 peut être enlevé, soit parce qu'il a traversé de part en part du dispositif 1 de pincement de fermeture si le passage de sectionnement 22 est traversant, soit par un mouvement de coulissement dans le sens opposé au sens d'introduction, si le passage de sectionnement 22 est non traversant, borgne.

Dans cette situation, il est alors possible de dissocier les parties 4a et 4b d'une part et les parties 5a et 5b d'autre part, en dissociant les uns des autres les moyens d'association 24a et 24 b par un mouvement de coulissement axial d'écartement (phase de dissociation). Toutefois, on laisse les parties 4a et 5a d'une part, 4b et 5b d'autre part fixées aux parties extrêmes sectionnées du tube T en les pinçant et en les fermant.

On obtient alors un tube T sectionné en deux tronçons ou longueurs, chacun pincé et fermé à sa partie extrême sectionnée, à laquelle est fixée à cette fin une couple de parties, respectivement 4a et 5a d'une part, 4b et 5b d'autre part.

Ou, comme indiqué, on obtient un tube T sectionné en deux tronçons ou longueurs, qui d'un seul côté est pincé et fermé.

Selon une variante de réalisation, le passage de pincement 23 est d'ouverture réglable pour s'adapter à des tubes T de différentes géométries, notamment tailles.

A cet effet, il peut être prévu dans une réalisation de disposer de différents dispositifs 1 correspondant à des géométries, notamment tailles, différentes, ce qui permet de couvrir un large spectre de géométries de tubes T.

Dans une autre réalisation (figure 2), il est prévu que le mouvement relatif de rapprochement et de fermeture des première et seconde sections 3a et 3b est plus ou moins grand, de sorte que le passage de pincement 23 est, respectivement, plus ou moins petit. Cela est rendu possible par suite de l'existence de moyens de verrouillage réciproque 25a et 25b, comprenant une série de dents d'accrochage profilées étagées entre l'âme 7a, 7b de la section 3a, 3b et le bord libre de chant de la rive 8a, 8b attenante, ce qui permet par un anti retour, différentes positions relatives de verrouillage des sections 3a et 3b.

Dans une réalisation (figure 5), il est prévu que l'une ou/et l'autre des deux empreintes 11a et 11 b est légèrement inclinée le long de l'axe 6, vers le bord libre de chant de la rive 8a, 8b et vers l'extrémité proximale 20a, 20b, 21 a, 21 b.

Cette disposition constructive permet d'exercer un pincement du tube T plus important vers cette extrémité proximale 20a, 20b, 21 a, 21 b, ce qui est de nature à expulser le fluide se trouvant dans le tube T vers l'autre extrémité, distale 19.

Selon une réalisation (figure 7), les moyens d'association 24a et 24b comprennent une saillie 24a et une rainure 24b, ménagées sur les faces d'extrémité et proximales 20a, 20b, 21a, 21b, s'étendent non parallèlement à l'axe 6 comme dans la réalisation des figures 3 et 6, mais s'étendant transversalement. Dans ce cas, le mouvement d'association ou de dissociation est un mouvement de coulissement transversal parallèlement à la direction de la saillie 24a et de la rainure 24b, et non un mouvement axial.

Selon une autre réalisation (figure 8), les moyens d'association 27 diffèrent des moyens d'association à saillie 24a et trou ou rainure 24b, comme décrit précédemment, et comprennent une pièce amovible de liaison en forme de clip 27, apte à associer de façon amovible les deux parties 4a et 4b ou 5a et 5b dé façon non pas directe mais indirecte.

Ce clip 27 comprend une âme plate 28, apte à venir, lorsque le clip est monté en situation d'association, contre la face extérieure du dispositif 1 de pincement de fermeture. Cette âme 28 est percée d'une fente 26a apte à venir à l'aplomb de l'accès 26 et à se superposer à lui.

De l'âme 28 sont attenants quatre (ou un plus grand nombre) saillies en forme de tige 29a, profilées, parallèles entre elles et orthogonales à l'âme 28. Ces saillies 29a sont aptes à coopérer avec un nombre égal de rainures 29b, transversales, ménagées sur la face extérieure du dispositif 1 de pincement de fermeture, sur chaque partie 4a, 4b, 5a, 5b.

Dans les différentes réalisations précédemment décrites, les deux parties 4a et 5a d'une part, 4b et 5b d'autre part, des deux sections 3a et 3b, sont des pièces distinctes et le mouvement relatif de fermeture est un mouvement de coulissement transversal en translation.

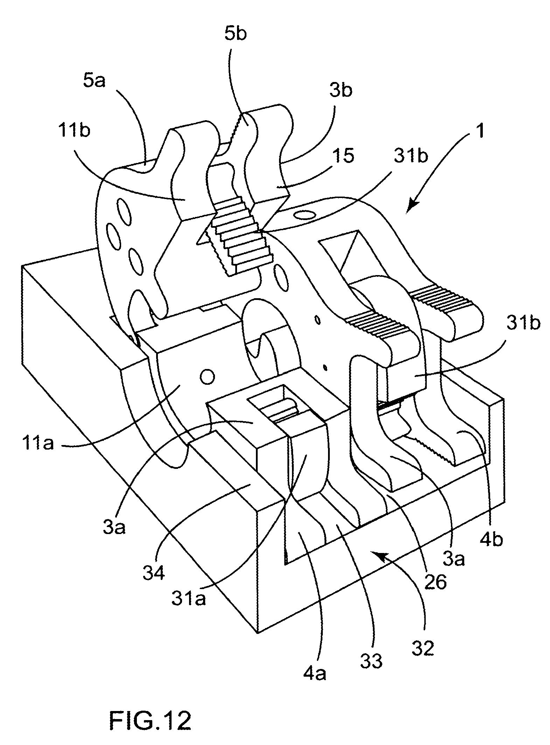

Dans une autre réalisation (figures 11 et 12), les deux parties 4a et 5a d'une part, 4b et 5b d'autre part, des deux sections 3a et 3b, sont des pièces non pas distinctes mais associées structurellement l'une à l'autre à pivotement autour d'un axe de pivotement 30 parallèle à l'axe 6 et le mouvement relatif de fermeture n'est pas un mouvement de coulissement transversal en translation mais un mouvement de pivotement autour de l'axe de pivotement 30. L'axe de pivotement 30 est déporté très largement par rapport à l'axe 6, sur un côté longitudinal du dispositif 1 de pincement de fermeture, de sorte que, le rayon de pivotement étant important, la fin du mouvement de pivotement d'emboîtement diffère peu d'un coulissement transversal, comme précédemment.

Du côté longitudinal opposé à l'axe 6, il est prévu des moyens de verrouillage 25a et 25b du type comportant, pour l'un, une pièce femelle 31 a, rapportée sur l'une des parties 4a, 5a ou 4b, 5b, et pourvue de dents 25a et, pour l'autre, d'une pièce mâle 31b, rapportée sur l'autre des parties 5a, 4a ou 5b, 4b, et pourvue de dents 25b.

Les dents 25a, 25 b, comme précédemment, sont des dents d'accrochage irréversible profilées, orientées selon deux directions opposées, s'étendant axialement.

Dans la réalisation des figures 11 et 12, comme dans la réalisation de la figure 8, les moyens d'association 32 comprennent, à l'instar de la pièce 27, une pièce amovible de liaison en forme de berceau 32, apte à associer de façon amovible les deux parties 4a et 4b ou 5a et 5b de façon non pas directe mais indirecte.

Cette pièce 32 comprend un berceau 33, apte à venir, lorsque la pièce 32 est montée en situation d'association, contre la face extérieure du dispositif 1 de pincement de fermeture, sur un côté de celui-ci, de manière à laisser libre l'autre côté ou est accessible le passage de sectionnement 22.

Le berceau 33 comporte deux rives 34 se faisant face, aptes à venir enserrer les parties 4a et 4b ou 5a et 5b par leurs faces extrêmes distales 19.

Dans les réalisations précédemment décrites d'un ensemble de pincement de fermeture et de sectionnement 1 + 2, le dispositif 1 de pincement de fermeture n'inclut pas structurellement le au moins un organe de coupe 2, celui-ci étant toutefois associé fonctionnellement.

Selon d'autres réalisations, le dispositif 1 de pincement de fermeture inclut structurellement et fonctionnellement au moins un organe de coupe 2. Par exemple, dans le cas de la figure 9, l'organe de coupe 2 forme une sorte de guillotine ayant en élévation une forme de U, est montée à coulissement dans son propre plan sur la face d'extrémité proximale 20a ou 20b de l'une des parties 4a, 4b pourvue de moyen de guidage appropriés. Dans cette réalisation, il est prévu un seul organe de coupe 2 ou deux organes de coupe sur les deux faces d'extrémité 20a et 20b. Dans cette réalisation, l'accès 26 sert au passage d'un organe de commande du mouvement de l'organe de coupe 2, tel qu'une sorte de lame.

## Revendications

1. Dispositif (1) de pincement d'un tube souple dans une région de pincement, comprenant deux sections (3a, 3b), chacune ayant une partie creuse (4a et 5a) à empreinte mâle (11 b) et femelle (11 b), respectivement, pouvant se trouver soit à l'état ouvert où elles sont écartées l'une de l'autre soit à l'état fermé où elles sont rapprochées et fermées l'une sur l'autre par des moyens de verrouillage réciproque (25a et 25b) et où les faces (13 et 14) des empreintes (11 a et 11 b) forment un passage (23) de pincement positif du tube,
**caractérisé par le fait que** :
- les sections (3a et 3b) sont profilées et s'étendent le long de l'axe longitudinal (6), la zone de pincement positif de fermeture (ZPF) s'étendant sur une certaine longueur axiale,
- l'empreinte mâle (11b) a en section droite transversale une forme de U avec une âme (37) et deux ailes (38a et 38b), l'empreinte femelle (11a) a en section droite transversale une forme de U avec une âme ((35) et deux ailes ((36a et 36b) et le passage (23) de pincement a en section droite transversale une forme de U avec une âme (41) et deux ailes (42a et 42b),
- les deux ailes en regard (36a, 38a et 36b, 38b) de chaque couple d'ailes en regard de l'empreinte mâle (11a) et de l'empreinte femelle (11b) vont en se rapprochant l'une de l'autre vers leurs extrémités (43) opposées aux âmes des empreintes (35 et 37), au moins dans la zone de ces extrémités (43) et chacune des deux ailes (42a et 42b) du passage (23) de pincement a, entre les faces des deux empreintes (11a et 11 b) une ouverture dont la largeur va en diminuant vers son extrémité opposée à l'âme (41) du passage (23) de pincement,
- la largeur de l'ouverture du passage (23) de pincement est au plus légèrement plus petite que le double de l'épaisseur de la paroi du tube souple à pincer,
- le dispositif (1) de pincement est à usage unique et remplit une fonction de fermeture du tube sur lui-même dans la région de pincement empêchant tout passage du fluide dans le tube.

2. Dispositif (1) de pincement de fermeture selon la revendication 1, **caractérisé par le fait que** les moyens de verrouillage (25a et 25b) sont irréversibles, les deux sections (3a et 3b) une fois amenées à l'état fermé étant maintenues dans cet état par les moyens de verrouillage (25a et 25b) sans pouvoir être dissociées et comprennent des dents d'accrochage profilées, orientées selon deux directions opposées.

3. Dispositif (1) de pincement de fermeture selon l'une quelconque des revendications 1 et 2, **caractérisé par le fait que** les deux sections (3a et 3b) sont deux pièces distinctes et sont amenées à l'état fermé par un mouvement relatif de coulissement transversal, l'une (3a) des deux sections (3a et 3b) étant une section mâle et l'autre (3b) étant une section femelle, la section mâle (3a) comportant et étant limitée extérieurement par une première âme (7a) et deux premières rives (8a), la section femelle (3b) comportant et étant limitée extérieurement par une seconde âme (7a) et deux secondes rives (8b).

4. Dispositif (1) de pincement de fermeture selon la revendication 3, **caractérisé par le fait que** l'écartement entre les deux faces extérieures (9) opposées des deux premières rives (8a) est en correspondance avec l'écartement entre les deux faces intérieures (10) en regard des deux secondes rives (8b), de manière à permettre, à l'état fermé du dispositif (1) de pincement de fermeture, l'emboîtement avec ajustement des deux sections (3a et 3b), la couple de faces extérieures (9) étant alors en contact et en appui sur la couple de faces intérieures (10), et par la fait que les moyens de verrouillage réciproque (25a et 25b) sont deux ensembles comprenant une série de dents ménagées respectivement sur les deux faces extérieures (9) des premières rives (8a) et les deux faces intérieures (10) des secondes rives (8b) dans leur zone d'emboîtement.

5. Dispositif (1) de pincement de fermeture selon l'une quelconque des revendications 1 et 2, **caractérisé par le fait que** les deux sections (3a et 3b) sont deux pièces associées structurellement l'une à l'autre à pivotement autour d'un axe (30) qui, par rapport à l'axe longitudinal du dispositif (1) de pincement de fermeture est déporté sur un côté longitudinal du dispositif (1) de pincement de fermeture, les deux sections (3a et 3b) étant amenées à l'état fermé par un mouvement relatif de pivotement autour de l'axe de pivotement.(30).

6. Dispositif (1) de pincement de fermeture selon la revendication 5, **caractérisé par** le fait les moyens de verrouillage réciproque (25a et 25b) comprennent des dents situées du côté opposé à l'axe, et en particulier comprennent une pièce femelle (31 a) rapportée sur l'une des parties (4a) et pourvue de dents (25a) et une pièce mâle (31b) rapportée sur l'autre des parties (5a) et pourvue de dents (25b).

7. Dispositif (1) de pincement de fermeture selon l'une quelconque des revendications 1 à 6, **caractérisé par le fait que** les deux sections (3a et 3b) sont de même longueur axiale.

8. Dispositif (1) de pincement de fermeture selon l'une quelconque des revendications 1 à 7, **caractérisé par le fait que** soit les deux empreintes (11a et 11b) ont une même forme, une même disposition et de mêmes dimensions, constantes et uniformes, d'une extrémité libre à l'autre, soit l'une ou/et l'autre des deux empreintes (11 a, 11 b) est légèrement inclinée le long de l'axe (6) du dispositif (1) de pincement de fermeture, vers le bord libre de chant de la rive (8a, 8b) et vers l'extrémité proximale, pour exercer un pincement du tube plus important vers l'extrémité proximale et expulser le fluide se trouvant dans le tube vers l'extrémité distale (19).

9. Dispositif (1) de pincement de fermeture selon l'une quelconque des revendications 1 à 8, **caractérisé par le fait que** le passage de pincement (23) est d'ouverture réglable, le mouvement relatif pour amener une section (3b) en regard d'une autre (3a) et le dispositif (1) de pincement de fermeture à l'état fermé étant plus ou moins grand, pour que le passage de pincement (23) soit respectivement, plus ou moins petit.

10. Dispositif (1) de pincement de fermeture selon la revendication 9, en ce qu'elle dépend de l'une quelconque des revendications 2 à 8, **caractérisé par le fait qu'**il comprend une série de dents d'accrochage (25a et 25b) étagées, permettant différentes positions relatives de verrouillage des sections (3a et 3b), pour que le passage de pincement (23) soit plus ou moins petit.

11. Dispositif (1) de pincement de fermeture selon l'une quelconque des revendications 1 10 11, **caractérisé en ce que** chaque section (3a et 3b) comporte deux parties creuses (4a, 4b et 5a, 5b) placées dans le prolongement l'une de l'autre par des moyens d'association (24a, 24b, 27, 32), une couple au moins (4a et 5a) des parties étant à empreintes (11a, 11b).

12. Dispositif (1) de pincement de fermeture selon la revendication 11, **caractérisé en ce que** les deux parties creuses (4a, 4b et 5a, 5b) de chaque section (3a et 3b) sont soit analogues, le dispositif (1) de pincement de fermeture assurant une fonction de fermeture du tube sur lui-même dans deux zones de pincement positif de fermeture, soit différentes, le dispositif (1) de pincement de fermeture assurant une fonction de fermeture du tube sur lui-même dans une seule zone de pincement positif de fermeture et étant seulement maintenu dans l'autre zone.

13. Dispositif (1) de pincement de fermeture selon l'une quelconque des revendications 11 et 12, **caractérisé en ce que** les moyens de verrouillage (25a et 25b) sont soit irréversibles pour les deux couples de parties creuses (4a et 5a, 4b et 5b), soit irréversibles pour l'une des couples de parties creuses (4a et 5a ou 4b et 5b) et réversibles pour l'autre (4b et 5b ou 4a et 5a).

14. Dispositif (1) de pincement de fermeture selon l'une quelconque des revendications 11 à 13, **caractérisé par le fait que** les moyens d'association (24a, 24b, 27, 32) comprennent au moins une saillie (24a) ménagé sur l'une des parties (4a, 4b, 5a, 5b), apte à coopérer de façon amovible avec au moins un trou borgne ou creux ou rainure (24b) complémentaires, ménagé sur l'autre partie de la même section (3a, 3b).

15. Dispositif (1) de pincement de fermeture selon l'une quelconque des revendications 11 à 13, **caractérisé par le fait que** les moyens d'association (27) comprennent au moins une pièce amovible de liaison en forme de clip (27), apte à associer de façon amovible les deux parties (4a et 4b ou 5a et 5b) de façon indirecte, le clip (27) comprenant une âme (28) de laquelle sont attenants quatre saillies en forme de tige (29a) aptes à coopérer avec quatre rainures (29b) transversales ménagées sur la face extérieure du dispositif (1) de pincement de fermeture, sur chaque partie (4a, 4b, 5a, 5b).

16. Dispositif (1) de pincement de fermeture selon l'une quelconque des revendications 11 à 13, **caractérisé par le fait que** les moyens d'association (32) comprennent une pièce amovible de liaison en forme de berceau (32), apte à associer de façon amovible les deux parties (4a et 4b ou 5a et 5b) de façon indirecte, le berceau (33) comportant deux rives (34) se faisant face, aptes à venir enserrer les parties (4a et 4b ou 5a et 5b) par leurs faces extrêmes distales (19).

17. Dispositif (1) de pincement de fermeture selon l'une quelconque des revendications 11 à 16, **caractérisé par le fait que** les parties creuses (4a, 4b et 5a, 5b) sont placées dans le prolongement l'une de l'autre en ménageant entre elles un passage médian (22) débouchant sur la face extérieure du dispositif par un accès (26), le passage de pincement positif de fermeture (23) comportant au moins un tronçon (23a) auquel est adjacent axialement un espace (23b) dans lequel débouche le passage (22) et où le tube est d'une part maintenu et d'autre part aplati avec un certain degré.

18. Dispositif (1) de pincement de fermeture selon la revendication 17, **caractérisé par le fait que** l'accès (26) d'une part, le passage médian (22) et l'espace (23b) d'autre part, ont pour fonction, respectivement, de permettre d'une part l'introduction, d'autre part le passage d'un organe de coupe (2) du tube dans sa zone située dans l'espace (23b).

## Patentansprüche

1. Klemmvorrichtung (1) eines elastischen Schlauchs in einem Klemmbereich, der zwei Abschnitte enthält (3a, 3b), die beide einen Hohlraum (4a und 5a) mit männlicher (11b) und weiblicher (11b) Prägung enthalten, beziehungsweise sich in einem geöffneten Zustand, bei dem sie voneinander ausgebreitet sind, oder geschlossenen Zustand befinden, bei dem die sich aneinander annähern und durch ein gegenseitigen Verschlusssystem geschlossen sind (25a und 25b) und bei dem die Seiten (13 und 14) der Prägungen (11 a und 11 b) eine positive Klemmpassage (23) des Schlauchs formen, was durch den Tatbestand charakterisiert wird, dass:
- die Abschnitte (3a und 3b) sind profiliert und erstrecken sich über eine Längsschnittachse (6), die positive Klemmschließzone (KSZ) erstreckt sich über eine gewisse Länge der Achse,
- die männliche Prägung (11 b) hat in dem rechten, quer laufenden Abschnitt eine U-Form mit einem Kern (37) und zwei Flügeln (38a und 38b), die weibliche Prägung (11a) hat im rechten quer laufenden Abschnitt eine U-Form mit einem Kern (35) und zwei Flügeln (36a und 36b) und die Klemmpassage (23) hat in dem rechten quer laufenden Abschnitt eine U-Form mit einem Kern (41) und zwei Flügeln (42a und 42b),
- die beiden gegenüberliegenden Flügel (36a, 38a und 36b, 38b) und jedes gegenüberliegende Flügelpaar mit männlicher Prägung (11a) und weiblicher Prägung (11b) bewegen sich auf einander in Richtung ihrer den Prägungskernen (35 und 37) gegenüberliegenden Enden (43) zu mindestens in diesen Endbereichen (43) zu und jeder der beiden Flügel (42a und 42b) der Klemmpassage (23) hat, zwischen den Oberflächen der beiden Prägungen (11a und 11 b) eine Öffnung, deren Breite sich von den gegenüberliegenden Enden zum Kern (41) der Klemmpassage (23) hin verringert,
- die Breite der Öffnung der Klemmpassage (23) ist höchstens etwas kleiner wie die doppelte Dicke der zu klemmenden elastischen Schlauchwand,
- die Klemmvorrichtung (1) ist zum einmaligen Gebrauch bestimmt und erfüllt die Funktion eines Selbstverschlusses des Schlauchs in dem Klemmbereich, was jeden Flüssigkeitsfluss im Schlauch verhindert.

2. Klemmvorrichtung (1) zum Verschließen entsprechend des Anspruchs 1, die durch den Tatbestand charakterisiert ist, dass die Verschlussmechanismen (25a und 25b) unumkehrbar sind, die beiden Abschnitte (3a und 3b) bleiben sobald sie in den verschlossenen Zustand versetzt worden sind durch ein Verschlusssystem (25a und 25b) in diesem Zustand, ohne dass sie voneinander abgekoppelt werden können und sie enthalten profilierte Rasterzähne, die in verschiedene Direktionen ausgerichtet sind.

3. Klemmvorrichtung (1) zum Verschließen gemäß irgendeinem der Ansprüche 1 und 2, die durch den Tatbestand charakterisiert ist, dass die beiden Abschnitte (3a und 3b) zwei unterschiedliche Stücke sind und durch eine relative Bewegung einer Querverschiebung zum verschlossenen Zustand gebracht werden, eine (3a) der beiden Abschnitte (3a und 3b) hat ein männlich Prägung und die Andere (3b) eine weibliche, der männliche Abschnitt (3a) beinhaltet und wird äußerlich durch einen ersten Kern (7a) und die beiden ersten Abgrenzungen (8a) begrenzt, die weibliche Prägung beinhaltet und wird äußerlich durch einen zweiten Kern (7a) und zwei zweite Abgrenzung (8b) begrenzt.

4. Klemmvorrichtung (1) zum Verschließen entsprechend des Anspruchs 3, die durch den Tatbestand charakterisiert ist, dass der Abstand zwischen den beiden äußeren Seiten (9) gegenüber den zwei ersten Abgrenzungen (8a) mit dem Abstand zwischen den beiden inneren Seiten (10) gegenüber den zwei zweiten Abgrenzungen (8b) dergestalt übereinstimmt, dass im geschlossene Zustand der Klemmvorrichtung (1) zum Verschließen das Fixieren mit der Anpassung der beiden Abschnitte (3a und 3b) möglich ist, das Paar der äußeren Seiten (9) ist also in Kontakt und in Unterstützung des Paars der inneren Seiten (10), wodurch erfolgt, dass die gegenseitigen Verschlussmechanismen (25a und 25b) zwei Zusammenhänge sind, die eine Reihe von bearbeiteten Zähnen enthalten in Bezug auf die beiden äußeren Seiten (9) der ersten Abgrenzung (8a) und die beiden inneren Seiten (10) der zweiten Abgrenzungen (8b) in ihrem Fixierbereich.

5. Klemmvorrichtung (1) zum Verschließen gemäß irgendeinem der Ansprüche 1 und 2, die durch den Tatbestand charakterisiert ist, dass die beiden Abschnitte (3a und 3b) zwei strukturell durch ein Schwenken über ein Achse (30) verbundene Teile sind, die im Verhältnis zur Längsachse der Klemmvorrichtung (1) zum Verschließen auf die Längsseite der Klemmvorrichtung (1) zum Verschließen bewegt wird, die beiden Abschnitte (3a und 3b) werden durch eine relative Schwenkbewegung um die Schwenkachse herum (30) zum geschlossenen Zustand gebracht.

6. Klemmvorrichtung (1) zum Verschließen gemäß des Anspruchs 5, die durch den Tatbestand charakterisiert ist, dass die gegenseitigen Verschlussvorkehrungen (25a und 25b) Zähne beinhalten, die sich auf der gegenüberliegenden Seite der Achse befinden und insbesondere ein weibliches Teil (31 a) beinhalten, das auf eines der Teile (4a) zurückgebracht wird und mit Zähnen versehen ist (25a) und ein männliches Teil (31 b), dass auf das andere der Teile zurückgebracht wird (5a) und mit Zähnen versehen ist (25b).

7. Klemmvorrichtung (1) zum Verschließen gemäß irgendeinem der Ansprüche 1 bis 6, die durch den Tatbestand charakterisiert ist, dass die beiden Abschnitte (3a und 3b) dieselbe Achsenlänge aufweisen.

8. Klemmvorrichtung (1) zum Verschließen gemäß irgendeinem der Ansprüche 1 bis 7, die durch den Tatbestand charakterisiert ist, dass die beiden Abdrücke (11 a und 11 b) entweder dieselbe Form, dieselbe Anordnung oder dieselben gleichmäßigen und einheitlichen Dimensionen von einem freien Ende zum anderen haben, oder das eine oder/und das andere der beiden Abdrücke (11 a, 11 b) leicht gebeugt entlang der Achse (6) der Klemmvorrichtung (1) zum Verschließen hin zum freien Ende der Profilseite (8a, 8b) und zum proximalen Endabschnitt ist, um eine besseres Verschließen des Schlauchs zum proximalen Endabschnitt auszuüben und die sich im Schlauch befindliche Flüssigkeit zum distalen Ende (19) hin auszustoßen.

9. Klemmvorrichtung (1) zum Verschließen gemäß irgendeiner der Ansprüche 1 bis 8, die durch den Tatbestand charakterisiert ist, dass die Klemmpassage (23) eine regulierbare Öffnung, die relative Bewegung, um einen Abschnitt (3b) in Verhältnis zu einem anderen (3a) zu bringen und die Klemmvorrichtung (1) zum Verschließen mit einem mehr oder wenig großen Verschlusszustand hat, damit die Klemmpassage (23) vergleichsweise mehr oder weniger klein ist.

10. Klemmvorrichtung (1) zum Verschließen gemäß des Anspruchs 9, soweit sie von einer der Ansprüche 2 bis 8 abhängt und von dem Tatbestand charakterisiert ist, dass sie eine Reihe von gestuften Rasterzähnen aufweist (25a und 25b), die die verschiedenen Positionen im Verhältnis zum Verschluss der Abschnitte (3a und 3b) erlaubt, damit die Klemmpassage (23) mehr oder weniger klein ist.

11. Klemmvorrichtung (1) zum Verschließen gemäß irgendeiner der Ansprüche 1 10 11, die dadurch charakterisiert ist, dass jeder Abschnitt (3a und 3b) zwei Hohlräume enthält (4a, 4b und 5a, 5b), die sich in der Verlängerung voneinander durch die Verbindungsmittel (24a, 24b, 27, 32) befinden, ein Paar oder weniger (4a und 5a) der Teile waren zu Abdrücken (11 a, 11 b) da.

12. Klemmvorrichtung (1) zum Verschließen gemäß des Anspruchs 11, die dadurch charakterisiert ist, dass die beiden Hohlräume (4a, 4b, und 5a, 5b) jedes Abschnitts (3a und 3b) entweder analog sind, die Klemmvorrichtung (1) zum Verschließen erfüllt die eigene Verschlussfunktion des Schlauchs in zwei positiven Klemmbereichen zum Verschließen oder unterschiedlich sind, die Klemmvorrichtung (1) zum Verschließen erfüllt die eigene Verschlussfunktion des Schlauchs in einem einzigen positiven Klemmbereich zum Verschließen und wird nur in dem anderen Bereich beibehalten.

13. Klemmvorrichtung (1) zum Verschließen gemäß irgendeiner der Ansprüche 11 und 12, die dadurch charakterisiert ist, dass die Schließmechanismen (25a und 25b) entweder für die beiden Paare der Hohlräume (4a und 5a, 4b und 5b) oder für eines der Paare der Hohlräume (4a und 5a oder 4b und 5b) unumkehrbar und umkehrbar für das Andere (4b und 5b oder 4a und 5a) sind.

14. Klemmvorrichtung (1) zum Verschließen gemäß irgendeiner der Ansprüche 11 bis 13, die durch den Tatbestand charakterisiert sind, dass die Verbindungsmittel (24a, 24b, 27, 32) mindestens eine Ausbuchtung (24a) über eine der Teile (4a, 4b, 5a, 5b) beinhalten, der in der Lage ist auf herausnehmbare Art mit mindestens einem zusätzlichen Sackloch oder Hohlraum oder Rille (24b) zusammenzuarbeiten, die am anderen Teil desselben Bereichs (3a, 3b) vorgesehen ist.

15. Klemmvorrichtung (1) zum Verschließen gemäß irgendeiner der Ansprüche 11 bis 13, die durch den Tatbestand charakterisiert ist, dass die Verbindungsmittel (27) zu mindestens ein herausnehmbares Verbindungsteil in Clipform (27) beinhalten, das in der Lage ist auf herausnehmbare Art die zwei Teile (4a und 4b oder 5a und 5b) auf indirekte Art zu verbinden, der Clip (27) beinhaltet einen Kern (28) an dem vier Ausbuchtungen in Stilform angrenzen (29a), die in der Lage sind mit den vier quer laufende Rinnen (29b) zusammenzuarbeiten, die auf der äußeren Seite der Klemmvorrichtung (1) zum Verschließen auf jedem Teil (4a und 4b oder 5a und 5b) vorgesehen sind.

16. Klemmvorrichtung (1) zum Verschließen gemäß irgendeiner der Ansprüche 11 bis 13, die durch den Tatbestand charakterisiert ist, dass die Verbindungsmittel (32) ein herausnehmbares Verbindungsteil in Wiegenform (32) beinhalten, die geeignet sind auf herausnehmbare Art die beiden Teile auf indirekte Weise zu verbinden (4a und 4b oder 5a und 5b), die Wiege (33) umfasst zwei Abgrenzungen (34), die sich gegenüberliegen und geeignet sind die Teile (4a und 4b oder 5a und 5b) über ihre äußersten Distalseiten (19) festzuzurren.

17. Klemmvorrichtung (1) zum Verschließen gemäß irgendeiner der Ansprüche 11 bis 16, die durch den Tatbestand charakterisiert ist, dass die Hohlräume (4a, 4b und 5a, 5b) sich in der Verlängerung voneinander befinden und dabei zwischen ihnen eine mittlere Passage (22) vorsehen, wodurch auf der Außenseite der Vorrichtung ein Zugang (26) entsteht, die positive Klemmpassage zum Verschließen (23) beinhaltet mindestens einen Abschnitt (23a), an den wie eine Achse ein Raum angrenzt (23b) zu dem die Passage (22) führt und wo der Schlauch einerseits aufrechterhalten und anderseits mit einen bestimmten Grad zugedrückt wird.

18. Klemmvorrichtung (1) zum Verschließen gemäß des Anspruchs 17, die durch den Tatbestand charakterisiert ist, dass der Zugang (26) auf der einen Seite, die mittlere Passage (22) und der Raum (23b) auf der anderen Seite die Funktion haben beziehungsweise sie erlauben einerseits die Einführung und andererseits die Passage des Schneidorgans (2) des Schlauchs in dem Bereich, der sich im Raum (23b) befindet.

## Claims

1. Device (1) for pinching a flexible tube in a pinching region, comprising two cross-sections (3a, 3b), each having a hollow part (4a, 5a) with a male indentation (11b) and a female indentation (11b), respectively, able to be either in the open state where they are separated from one another or in the closed state where they are drawn together and closed on one another by reciprocal-locking means (25a and 25b) and where the surfaces (13 and 14) of the indentations (11a and 11b) form a positive pinching passage (23) of the tube, **characterized by** the fact that:
- the cross-sections (3a and 3b) are contoured and extend along the longitudinal axis (6), with the positive pinching closing zone (ZPF) extending over a certain axial length,
- in the transverse straight cross-section, the male indentation (11b) has a U shape with a core (37) and two wings (38a and 38b); in the transverse straight cross-section, the female indentation (11a) has a U shape with a core (35) and two wings (36a and 36b); and in the transverse straight cross-section, the pinching passage (23) has a U shape with a core (41) and two wings (42a and 42b),
- the two wings (36a, 38a and 36b, 38b) opposite each pair of wings facing the male indentation (11a) and the female indentation (11b) will draw together toward their ends (43) that are opposite to the cores of the indentations (35 and 37), at least in the zone of these ends (43), and each of the two wings (42a and 42b) of the pinching passage (23) has, between the surfaces of the two indentations (11a and 11b), an opening whose width will diminish towards its end that is opposite to the core (41) of the pinching passage (23),
- the width of the opening of the pinching passage (23) is at most slightly smaller than twice the thickness of the wall of the flexible tube to be pinched,
- the pinching device (1) is disposable and performs an operation of the tube closing on itself in the pinching region preventing any passage of fluid into the tube.

2. Pinching closing device (1) according to Claim 1, **characterized by** the fact that the locking means (25a and 25b) are irreversible, with the two cross-sections (3a and 3b) - once brought into the closed state - being held in place in this state by locking means (25a and 25b) without being able to be dissociated, and comprise contoured hooking teeth, oriented in two opposite directions.

3. Pinching closing device (1) according to any of Claims 1 and 2, **characterized by** the fact that the two cross-sections (3a and 3b) are two separate parts and are brought to the closed state by a relative transverse sliding movement, whereby one (3a) of the two cross-sections (3a and 3b) is a male cross-section and the other (3b) is a female cross-section, whereby the male cross-section (3a) comprises and is limited on the outside by a first core (7a) and two first lips (8a), with the female cross-section (3b) comprising and being limited on the outside by a second core (7a) and two second lips (8b).

4. Pinching closing device (1) according to Claim 3, **characterized by** the fact that the separation between the two opposite outside surfaces (9) of the two first lips (8a) corresponds to the separation between the two inside surfaces (10) opposite the two second lips (8b), in such a way as to allow, in the closed state of the pinching closing device (1), the interlocking with adjustment of the two cross-sections (3a and 3b), with the pair of outside surfaces (9) then being in contact and supported on the pair of inside surfaces (10), and wherein the reciprocal-locking means (25a and 25b) are two units that comprise a series of teeth respectively made on the two outside surfaces (9) of the first lips (8a) and the two inside surfaces (10) of the second lips (8b) in their interlocking zone.

5. Pinching closing device (1) according to any of Claims 1 and 2, **characterized by** the fact that the two cross-sections (3a and 3b) are two parts that are structurally combined with one another to pivot around an axis (30) that, relative to the longitudinal axis of the pinching closing device (1), is offset on a longitudinal side of the pinching closing device (1), with the two cross-sections (3a and 3b) being brought into the closed state by a relative pivoting movement around the pivoting axis (30).

6. Pinching closing device (1) according to Claim 5, **characterized by** the fact that the reciprocal-locking means (25a and 25b) comprise teeth that are located on the side opposite to the axis and in particular comprise a female part (31a) that is connected to one of the parts (4a) and equipped with teeth (25a) and a male part (31b) that is connected to the other part (5a) and equipped with teeth (25b).

7. Pinching closing device (1) according to any of Claims 1 to 6, **characterized by** the fact that the two cross-sections (3a and 3b) have the same axial length.

8. Pinching closing device (1) according to any of Claims 1 to 7, **characterized by** the fact that the two indentations (11a and 11b) have the same shape, the same arrangement, and the same dimensions, constant and uniform, from one free end to the next, or one and/or the other of the two indentations (11a, 11b) is slightly inclined along the axis (6) of the pinching closing device (1), toward the free border of the edge of the lip (8a, 8b) and toward the proximal end, for exerting a more significant pinching of the tube toward the proximal end and for expelling the fluid that is in the tube toward the distal end (19).

9. Pinching closing device (1) according to any of Claims 1 to 8, **characterized by** the fact that the pinching passage (23) has an adjustable opening, with the relative movement for bringing one cross-section (3b) opposite the other (3a) and the pinching closing device (1) in the closed state being more or less large, so that the pinching passage (23) is respectively more or less small.

10. Pinching closing device (1) according to Claim 9, in that it depends on any of Claims 2 to 8, **characterized by** the fact that it comprises a series of stepped hooking teeth (25a and 25b), making possible different relative locking positions of the cross-sections (3a and 3b) so that the pinching passage (23) is more or less small.

11. Pinching closing device (1) according to any of Claims 1 to 10, **characterized by** the fact that each cross-section (3a and 3b) comprises two hollow parts (4a, 4b and 5a, 5b) that are placed in the extension of one another by combination means (24a, 24b, 27, 32), with at least one pair (4a and 5a) of parts having indentations (11a, 11b).

12. Pinching closing device (1) according to Claim 11, **characterized by** the fact that the two hollow parts (4a, 4b and 5a, 5b) of each cross-section (3a and 3b) are either analogous, with the pinching closing device (1) ensuring a closing function of the tube on itself in two positive pinching closing zones, or different, with the pinching closing device (1) ensuring a closing function of the tube on itself in a single positive pinching closing zone and being only held in place in the other zone.

13. Pinching closing device (1) according to any of Claims 11 and 12, **characterized by** the fact that the locking means (25a and 25b) are either irreversible for the two pairs of hollow parts (4a and 5a, 4b and 5b), or irreversible for one of the pairs of hollow parts (4a and 5a or 4b and 5b) and reversible for the other (4b and 5b or 4a and 5a).

14. Pinching closing device (1) according to any of Claims 11 to 13, **characterized by** the fact that the combination means (24a, 24b, 27, 32) comprise at least one projection (24a) made on one of the parts (4a, 4b, 5a, 5b), able to work in a removable way with at least one blind hole or hollow or groove (24b) - complementary and made on the other part of the same cross-section (3a, 3b).

15. Pinching closing device (1) according to any of Claims 11 to 13, **characterized by** the fact that the combination means (27) comprise at least one removable connecting part in the form of a clip (27), able to combine the two parts (4a and 4b or 5a and 5b) in a removable way and in an indirect way, with the clip (27) comprising a core (28) from which four projections that are in rod form (29a) and that are able to work with four transverse grooves (29b) made on the outer surface of the pinching closing device (1) are adjacent, on each part (4a, 4b, 5a, 5b).

16. Pinching closing device (1) according to any of Claims 11 to 13, **characterized by** the fact that the combination means (32) comprise a removable connecting part in the shape of a cradle (32), able to combine the two parts (4a and 4b or 5a and 5b) in a removable way and in an indirect way, with the cradle (33) comprising two lips (34) that face one another, able to encircle the parts (4a and 4b or 5a and 5b) by their distal end surfaces (19).

17. Pinching closing device (1) according to any of Claims 11 to 16, **characterized by** the fact that the hollow parts (4a, 4b and 5a, 5b) are placed in the extension of one another by making between them a median passage (22) that empties onto the outside surface of the device by an access (26), whereby the positive pinching closing passage (23) comprises at least one segment (23a) to which a space (23b) - into which the passage (22) empties and where the tube is held in place, on the one hand, and flattened to a certain degree, on the other hand - is axially adjacent.

18. Pinching closing device (1) according to Claim 17, **characterized by** the fact that the functions of the access (26), on the one hand, and the median passage (22), and the space (23b), on the other hand, are respectively to allow, on the one hand, the introduction, and, on the other hand, the passage of an element (2) for cutting the tube in its zone located in said space (23b).
